# EUROPEAN PATENT APPLICATION

(11) **EP 4 765 160 A1**
(43) Date of publication of application: **24.06.2026**
(21) Application number: 25767368.1
(22) Date of filing: 04.03.2025
(51) Int. Cl.: G16H 80/00

(54) **PHYSICAL INDICATOR MANAGEMENT METHOD, USER INTERFACE, AND RELATED APPARATUS**

(30) Priority: 06.03.2024 CN 202410260479
(71) Applicant: HUAWEI TECHNOLOGIES CO., LTD., Shenzhen 518129 (CN)
(72) Inventor: YANG, Yantao, Shenzhen, Guangdong 518129 (CN); LIU, Xiangyu, Shenzhen, Guangdong 518129 (CN); LI, Xiaodong, Shenzhen, Guangdong 518129 (CN); PAN, Jun, Shenzhen, Guangdong 518129 (CN); XIE, Songlin, Shenzhen, Guangdong 518129 (CN)
(74) Representative: Pfenning, Meinig & Partner mbB
(86) International application number: PCT/CN2025/080539
(87) International publication number: WO 2025/185630

(57) **Abstract**

A body indicator management method, a user interface, and a related apparatus are disclosed. In the method, a first device may obtain first data, where the first data indicates a status of a body indicator of a user monitored by a second device at first time; the first device obtains second data, where the second data indicates a status of the body indicator of the user monitored by a third device at the first time; and the first device may identify accuracy of the first data or the second data based on the first data and the second data. In this way, accurate data and inaccurate data that are collected by devices configured to monitor the body indicator can be distinguished, so that the user can learn about an actual and accurate status of the body indicator of the user.

## Description

This application claims priority to Chinese Patent Application No. 202410260479.6, filed with the China National Intellectual Property Administration on March 6, 2024, and entitled "BODY INDICATOR MANAGEMENT METHOD, USER INTERFACE, AND RELATED APPARATUS", which is incorporated herein by reference in its entirety.

### TECHNICAL FIELD

This application relates to the field of terminal and computer technologies, and in particular, to a body indicator management method, a user interface, and a related apparatus.

### BACKGROUND

With continuous progress of science and technology, an increasing quantity of devices are designed to be portable and worn by a user, and are configured to measure a body indicator of the user, including blood glucose, a heart rate, a body temperature, blood pressure, blood lipid, blood ketone, pondus hydrogenii (pondus hydrogenii, PH), creatinine, uric acid, and the like. For example, common devices include: a continuous glucose monitoring (continuous glucose monitoring, CGM) device, where the device may be configured to measure blood glucose of the user; a continuous ketone monitoring (continuous ketone monitoring, CKM) device, where the device may be configured to measure blood ketone of the user; and a continuous lactate monitoring (continuous lactate monitoring, CLM) device, where the device may be configured to measure lactate of the user.

However, when this type of device is worn by the user, various factors such as a collision with an external object and an impact of an immune response of the user lead to inaccurate output values of the device, thereby affecting evaluation of the body indicator of the user.

### SUMMARY

This application provides a body indicator management method, a user interface, and a related apparatus, to identify, through a plurality of devices that are worn by a user and that are configured to monitor a body indicator, accuracy of body indicator data collected by the plurality of devices.

According to a first aspect, an embodiment of this application provides a body indicator management method. The method includes: A first device obtains first data, where the first data indicates a status of a body indicator of a user monitored by a second device at first time; the first device obtains second data, where the second data indicates a status of the body indicator of the user monitored by a third device at the first time; and the first device identifies accuracy of the first data or the second data based on the first data and the second data.

According to the method provided in the first aspect, a case in which a body indicator collected by a device is inaccurate due to impact of various factors can be considered, and accuracy of body indicator data collected by one of a plurality of devices is identified by using body indicator data collected by the plurality of devices, so that the user can have a clearer and more accurate cognition of a physical condition of the user based on the body indicator data collected by the devices.

With reference to the first aspect, in an implementation, after the first device identifies the accuracy of the first data or the second data based on the first data and the second data, the method further includes: The first device outputs first prompt information, where the first prompt information is used to give the user a prompt that the first data or the second data is inaccurate.

If the electronic device identifies inaccurate body indicator data, the electronic device may output prompt information, so that the user can distinguish between accurate body indicator data and inaccurate body indicator data that are collected by the devices.

With reference to the first aspect, in an implementation, the method further includes: The first device displays a first curve, where the first curve includes any one or more of the following: a curve segment drawn based on the first data, a curve segment drawn based on the second data, or a curve segment drawn based on the first data and the second data.

In other words, the electronic device may display body indicator data collected by a body indicator monitoring device, so that the user learns about a monitoring status of the body indicator monitoring device for the body indicator of the user, and learns about a fluctuation status of the body indicator of the user.

With reference to the first aspect, in an implementation, the first time includes a first time period and a second time period, data in the first time period and data in the second time period that are included in the first data are accurate, data in the first time period included in the second data is inaccurate, and data in the second time period included in the second data is accurate. The curve segment drawn based on the first data and the second data includes: a curve segment drawn based on the data in the first data in the first time period, and a curve segment drawn based on third data, where the third data is the data in the first data or the second data in the second time period, or the third data is data determined based on the data in the first data and the data in the second data in the second time period.

In other words, if the electronic device displays a curve drawn based on the body indicator data, the electronic device may display only a curve drawn based on accurate data, to avoid impact of inaccurate data on evaluation of the user on the body indicator of the user.

With reference to the first aspect, in an implementation, the first time includes the first time period and the second time period, the data in the second data in the first time period is inaccurate, the data in the second data in the second time period is accurate, and after the first device identifies the accuracy of the first data or the second data based on the first data and the second data, the method further includes: The first device displays a first curve drawn based on the second data, where the first curve includes a curve segment corresponding to the second time period, and does not include a curve segment corresponding to the first time period, or the first curve includes a first curve segment corresponding to the first time period and a second curve segment corresponding to the second time period, and a display effect of the first curve segment and a display effect of the second curve segment are different.

In other words, when displaying a curve drawn based on body indicator data collected by a device, the electronic device may display accurate data, and does not display inaccurate data, or accurate data and inaccurate data may be displayed separately. In this way, the inaccurate data can be prevented from misleading the user to evaluate the body indicator of the user, so that the user has a clearer and more accurate cognition of the body condition of the user.

With reference to the first aspect, in an implementation, the first curve includes the curve segment corresponding to the second time period, and does not include the curve segment corresponding to the first time period; and after the first device displays the first curve drawn based on the second data, the method further includes: The first device detects a first operation, and updates the first curve, where the updated first curve includes the curve segment corresponding to the first time period and the curve segment corresponding to the second time period.

In other words, the electronic device may choose, based on a user operation, whether to display the inaccurate data, thereby improving operability of the user.

With reference to the first aspect, in an implementation, the first device further displays first exception information, and the first exception information indicates a reason why the data in the second data in the first time period is inaccurate.

The electronic device may display a reason why the body indicator is inaccurate, so that the user can learn about, from a plurality of aspects, a device status or the body condition of the user when the body indicator monitoring device collects the body indicator data.

With reference to the first aspect, in an implementation, after the first device identifies the accuracy of the first data or the second data based on the first data and the second data, the method further includes: The first device evaluates the body indicator of the user based on fourth data, where the fourth data includes accurate data in the first data and the accurate data in the second data, and does not include inaccurate data in the first data and the inaccurate data in the second data.

In other words, the electronic device may accurately evaluate the status of the body indicator of the user based on accurate data.

With reference to the first aspect, in an implementation, before obtaining, by the first device, the first data, the first device establishes a communication connection to the second device, and the method further includes: The first device outputs second prompt information at second time, where the second prompt information is used to prompt the user to wear a device configured to monitor the body indicator, and a first preset duration is to elapse since the second time until the second device expires.

It can be learned that the electronic device can remind the user in time to wear a new body indicator monitoring device before a body indicator monitoring device expires.

With reference to the first aspect, in an implementation, the first preset duration is greater than a first duration, and the first duration indicates an initialization duration of the device configured to monitor the body indicator.

A CGM device is used as an example. Because after a probe of the CGM device is implanted under skin, an electrochemical reaction needs to be generated between the probe and glucose in tissue fluid, and a current formed by electronic directional movement is used to reflect a blood glucose value of the user, after the CGM device is worn, the user needs to wait for a period of time, for example, half an hour or one hour, for completing device initialization, and then, the CGM device can collect a stable and accurate blood glucose value. Therefore, a duration for the CGM device to complete initialization is referred to as the initialization duration.

In other words, the electronic device may remind, before one initialization duration before a body indicator monitoring device expires, the user to wear a new body indicator monitoring device, so that the electronic device can continuously monitor the status of the body indicator of the user.

With reference to the first aspect, in an implementation, before obtaining, by the first device, the first data, the method further includes: After establishing the communication connection to the second device, the first device outputs, after a second duration, data that is collected by the second device and that is used to reflect the body indicator of the user, where the second duration is determined based on the first duration and a duration from starting to wear the second device to establishing the communication connection to the first device by the second device, and the first duration indicates the initialization duration of the device configured to monitor the body indicator.

In other words, the electronic device may consider a duration elapsed since the user wears the body indicator monitoring device, to shorten a countdown duration of the electronic device as much as possible, shorten waiting time of the user, and output the body indicator data collected by the body indicator monitoring device as soon as possible.

With reference to the first aspect, in an implementation, wearing time of the second device is earlier than wearing time of the third device, the first time is in a first preset time period after the third device starts to be worn, and the first device identifies the accuracy of the second data based on the first data and the second data. If a probability that early sensitivity attenuation ESA occurs on the second device when the second device collects the first data is less than a first threshold, and a probability that early sensitivity attenuation occurs on the third device when the third device collects the second data is greater than a second threshold, the second data is inaccurate; or if a probability that early sensitivity attenuation occurs on the third device when the third device collects the second data is greater than a third threshold, the second data is inaccurate, where the probability that the early sensitivity attenuation occurs on the third device when the third device collects the second data is determined based on a probability that early sensitivity attenuation occurs on the second device when the second device collects the first data.

When early sensitivity attenuation occurs on the body indicator monitoring device, the body indicator data collected by the body indicator monitoring device is inaccurate. Therefore, the electronic device may identify, with reference to wearing time of the device and the body indicator data collected by the device, whether early sensitivity attenuation occurs on the body indicator device, and further identify accuracy of the body indicator data.

With reference to the first aspect, in an implementation, the first preset time period is preset by a developer or determined by the first device based on historically obtained body indicator data.

In other words, a time period in which the body indicator monitoring device is prone to early sensitivity attenuation may be preset by the developer, or may be obtained through calculation based on the historical body indicator data.

With reference to the first aspect, in an implementation, the wearing time of the second device is earlier than the wearing time of the third device, the first time is in a second preset time period before the second device expires, and the first device identifies the accuracy of the first data based on the first data and the second data. If a probability that late sensor attenuation LSA occurs on the second device when the second device collects the first data is greater than a fourth threshold, and a probability that late sensor attenuation occurs on the third device when the third device collects the second data is less than a fifth threshold, the first data is inaccurate; or if a probability that late sensor attenuation occurs on the second device when the second device collects the first data is greater than a sixth threshold, the first data is inaccurate, where the probability that the late sensor attenuation occurs on the second device when the second device collects the first data is determined based on a probability that late sensor attenuation occurs on the third device when the third device collects the second data.

When late sensor attenuation occurs on the body indicator monitoring device, the body indicator data collected by the body indicator monitoring device is inaccurate. Therefore, the electronic device may identify, with reference to the wearing time of the device and the body indicator data collected by the device, whether late sensor attenuation occurs on the body indicator device, and further identify accuracy of the body indicator data.

With reference to the first aspect, in an implementation, the second preset time period is preset by the developer or determined by the first device based on the historically obtained body indicator data.

In other words, a time period in which the body indicator monitoring device is prone to late sensor attenuation may be preset by the developer, or may be obtained through calculation based on the historical body indicator data.

With reference to the first aspect, in an implementation, that the first device identifies the accuracy of the first data or the second data based on the first data and the second data specifically includes: If the first device identifies, based on the first data and the second data, that the second device is squeezed at the first time, the first device determines that the first data is inaccurate.

When the body indicator monitoring device is squeezed, the body indicator data collected by the body indicator monitoring device is inaccurate. Therefore, the electronic device may identify, based on the data collected by the device, whether the body indicator monitoring device is squeezed, and further identify accuracy of the body indicator data.

With reference to the first aspect, in an implementation, when the second device is squeezed at the first time, the first data has a fluctuation trend of first decreasing and then increasing.

If the body indicator monitoring device is squeezed, the body indicator data collected by the body indicator monitoring device presents a fluctuation trend of first decreasing and then increasing. Therefore, the electronic device may identify, by identifying whether the fluctuation trend of the body indicator data is first decreasing and then increasing, whether the body indicator monitoring device is squeezed.

With reference to the first aspect, in an implementation, the second device and the third device each are a continuous glucose monitoring CGM device, and the body indicator is blood glucose; or the second device and the third device each are a continuous ketone monitoring CKM device, and the body indicator is blood ketone; or the second device and the third device each are a continuous lactate monitoring CLM device, and the body indicator is lactate.

According to a second aspect, an embodiment of this application provides an electronic device, including a memory, one or more processors, and one or more programs. When the one or more processors execute the one or more programs, the electronic device is enabled to implement the method according to any one of the first aspect or the implementations of the first aspect.

According to a third aspect, an embodiment of this application provides a computer-readable storage medium including instructions. When the instructions are run on an electronic device, the electronic device is enabled to perform the method according to any one of the first aspect or the implementations of the first aspect.

According to a fourth aspect, an embodiment of this application provides a computer program product including a computer program. When the computer program is executed by a processor, the method according to any one of the first aspect or the implementations of the first aspect is implemented.

### BRIEF DESCRIPTION OF DRAWINGS

FIG. 1 is a diagram of a communication system 1000 according to an embodiment of this application;
FIG. 2A to FIG. 2E(c) and FIG. 3A to FIG. 3F show some user interfaces according to an embodiment of this application;
FIG. 4 shows a blood glucose change curve collected by an electronic device 200 after the electronic device 200 is worn by a user according to an embodiment of this application;
FIG. 5 is a schematic flowchart of an initialization method according to an embodiment of this application;
FIG. 6 is a schematic flowchart of a body indicator management method according to an embodiment of this application;
FIG. 7 is a diagram of a hardware structure of an electronic device 100 according to an embodiment of this application;
FIG. 8 is a block diagram of a software structure of an electronic device 100 according to an embodiment of this application;
FIG. 9 is a diagram of a hardware structure of an electronic device 200 according to an embodiment of this application; and
FIG. 10 is a diagram of a structure of a body indicator management apparatus 400 according to an embodiment of this application.

### DESCRIPTION OF EMBODIMENTS

The technical solutions according to embodiments of this application are clearly and completely described in the following with reference to the accompanying drawings. In the descriptions of embodiments of this application, unless otherwise stated, "/" represents "or". For example, A/B may represent A or B. In this specification, "and/or" merely describes an association relationship between associated objects and represents that three relationships may exist. For example, A and/or B may represent the following three cases: Only A exists, both A and B exist, and only B exists. In addition, in the descriptions of embodiments of this application, "a plurality of' means two or more.

The following terms "first" and "second" are merely intended for a purpose of description, and shall not be understood as an indication or implication of relative importance or implicitly indicating a quantity of indicated technical features. Therefore, a feature limited by "first" or "second" may explicitly or implicitly include one or more features. In the descriptions of embodiments of this application, unless otherwise specified, "a plurality of" means two or more.

A term "user interface (user interface, UI)" in the following embodiments of this application is a medium interface for interaction and information exchange between an application or an operating system and a user, and implements conversion between an internal form of information and a form that can be accepted by the user. The user interface is source code written in a specific computer language like Java or an extensible markup language (extensible markup language, XML). Interface source code is parsed and rendered on an electronic device, and is finally presented as content that can be identified by the user. A frequently-used representation form of the user interface is a graphical user interface (graphical user interface, GUI), and is a user interface that is displayed in a graphical manner and that is related to a computer operation. The user interface may be a visual interface element like a text, an icon, a button, a menu, a tab, a text box, a dialog box, a status bar, a navigation bar, or a widget that is displayed on a display of the electronic device.

Embodiments of this application provide a body indicator management method. The method relates to a first device, a second device, and a third device. The first device may obtain first data and second data, where the first data indicates a status of a body indicator of a user monitored by the second device at first time, the second data indicates a status of the body indicator of the user monitored by the third device at the first time, and the first device may identify accuracy of the first data or the second data based on the first data and the second data.

For example, the second device and the third device each may be a CGM device, and the body indicator may be blood glucose. Alternatively, the second device and the third device each may be a CKM device, and the body indicator may be blood ketone. Alternatively, the second device and the third device each may be a CLM device, and the body indicator may be lactate.

The second device and the third device may be devices worn by the user at different time.

In a case, in comparison with a device worn later, a device worn earlier may have an inaccurate output value due to factors such as long-term use of components and impact of an immune response of the user. Therefore, body indicator data collected by the device worn later by the user may be used to identify accuracy of body indicator data collected by the device worn earlier by the user.

In another case, in comparison with a device worn earlier, a device worn later may have an inaccurate output value due to factors such as unstable parameters at an early wearing stage and impact of an immune response of the user. Therefore, body indicator data collected by the device worn earlier by the user may be used to identify accuracy of body indicator data collected by the device worn later by the user.

In addition, the second device and the third device may be devices worn by the user at different parts.

If the different parts of the user respond differently to this type of device configured to monitor the body indicator, it is likely that some devices may collect body indicator data that does not reflect an actual body indicator of the user, or a part of a device worn on a body part of the user falls off due to impact of an external force, resulting in inaccuracy of body indicator data collected by the device. Therefore, body indicator data collected by devices worn by the user at different parts may be used to identify accuracy of data collected by each other.

For example, for the devices worn at the different parts, some devices may have an inaccurate output value due to squeezing of an external object or a limb of the user. Therefore, the body indicator data collected by the devices worn at the different parts may be used to identify accuracy of body indicator data collected by one of the devices worn by the user.

For example, after the first device identifies the accuracy of the body indicator data, the following application scenarios may exist:
(1) When displaying the body indicator data collected by the device, the first device displays, in a differentiated manner, body indicator data with different accuracy identified by the first device.
   In other words, the first device may not only display the body indicator data, but also display, in a differentiated manner, the body indicator data with different accuracy. In this way, the user can identify accurate data and inaccurate data collected by the device, so that the user can learn about an actual physical condition of the user.
(2) Select, based on the accuracy of the body indicator data identified by the first device, body indicator data used to evaluate the body indicator of the user

After obtaining the body indicator data, the first device may evaluate physical health of the user based on the body indicator data. After identifying the accuracy of the body indicator, the first device may remove inaccurate body indicator data, and evaluate a physical condition of the user based on accurate body indicator data, to provide an accurate physical evaluation result for the user.

It can be learned that, according to the body indicator management method provided in embodiments of this application, when the user wears a plurality of devices configured to monitor the body indicator, accuracy of body indicator data collected by one of the plurality of devices can be identified based on body indicator data collected by the plurality of devices, to avoid using false body indicator data collected by the device to evaluate the body indicator of the user.

It may be understood that the accuracy of the body indicator mentioned in embodiments of this application may also be replaced with another word, for example, validity or authenticity. For example, the accurate body indicator data may be valid body indicator data, and the inaccurate body indicator data may be invalid body indicator data. Therefore, descriptions of the accuracy in this specification does not constitute a limitation on embodiments of this application.

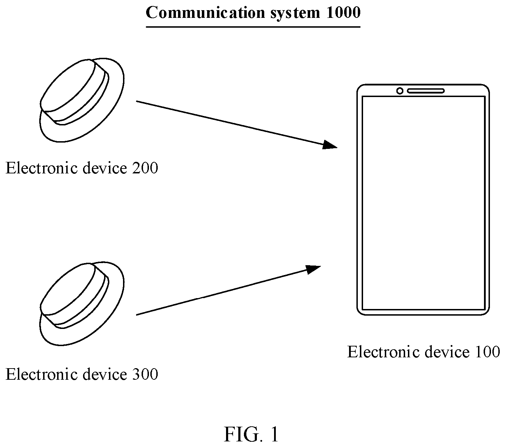

**FIG. 1** **is a diagram of a communication system 1000 according to an embodiment of this application.**

As shown in FIG. 1, the communication system 1000 may include an electronic device 100, an electronic device 200, and an electronic device 300.

The electronic device 200 and the electronic device 300 are devices worn by a user, and are configured to monitor a body indicator of the user. The device worn by the user may be a device worn externally on the user, for example, a watch or a band, or the device worn by the user may be a device that requires a component to be implanted into skin of the user, for example, a CGM device, a CKM device, or a CLM device.

The electronic device 100 may be configured to obtain body indicator data collected by the electronic device 200 and body indicator data collected by the electronic device 300. The body indicator data indicates a status of a body indicator of the user monitored by the device. In addition, the electronic device 100 may be further configured to identify, based on the body indicator data collected by the electronic device 200 and the body indicator data collected by the electronic device 300, accuracy of body indicator data collected by one (for example, the electronic device 200 or the electronic device 300) of the devices.

A communication connection is established between the electronic device 100 and each of the electronic device 200 and the electronic device 300. Specifically, the communication connection may be a wired connection or a wireless connection. The wireless connection may be a short-distance connection like a high-fidelity wireless communication (wireless fidelity, Wi-Fi) connection, a Bluetooth connection, an infrared connection, an NFC connection, or a ZigBee connection, or may be a long-distance connection. The long-distance connection includes but is not limited to a long-distance connection based on 2G, 3G, 4G, 5G, and mobile networks of subsequent standard protocols. For example, a Bluetooth connection may be established between the electronic device 100 and the electronic device 200, and the electronic device 100 may obtain, through the Bluetooth connection, the body indicator data collected by the electronic device 200. A Bluetooth connection is also established between the electronic device 100 and the electronic device 300, and the electronic device 100 may also obtain, through the Bluetooth connection, the body indicator data collected by the electronic device 300.

In this embodiment of this application, the electronic device 100 may also be referred to as a first device, the electronic device 200 may also be referred to as a second device, and the electronic device 300 may also be referred to as a third device.

It may be understood that the communication system 1000 may further include more devices. For example, the communication system 1000 may further include an electronic device 400. The electronic device 400 may obtain the body indicator data collected by the electronic device 200, and send the body indicator data to the electronic device 100. In addition, the communication system 1000 may further include more devices configured to monitor the body indicator. A quantity of devices included in the communication system 1000 is not limited in embodiments of this application.

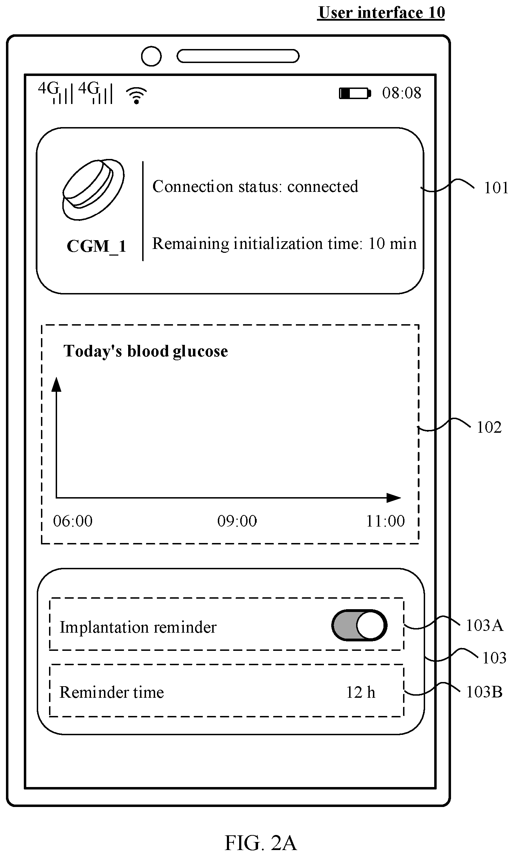

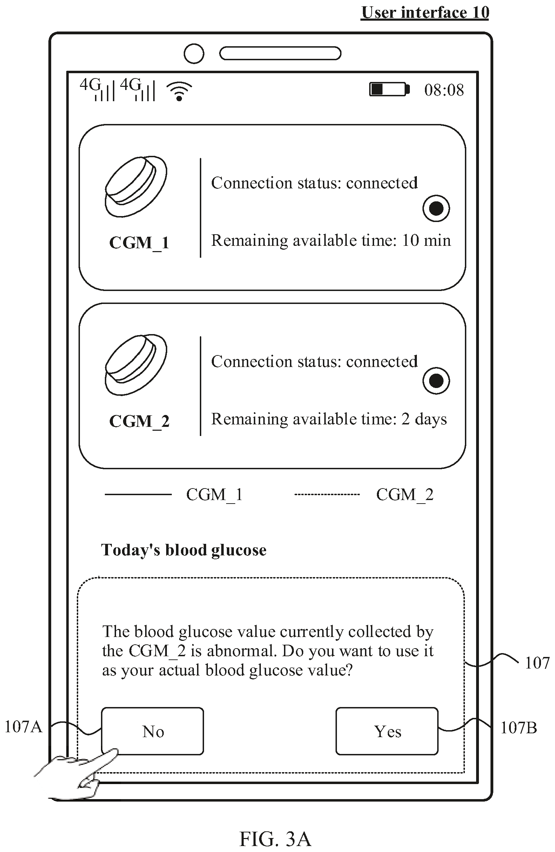

**The following uses an example in which the electronic device 200 and the electronic device 300 each are a CGM device to describe some user interfaces provided in embodiments of this application with reference to** **FIG. 2A to FIG. 2E(c)** **and** **FIG. 3A to FIG.** 3F.

FIG. 2A shows a user interface 10 that is displayed by the electronic device 100 after the electronic device 100 establishes the communication connection to the electronic device 200 and that is used to display information related to the electronic device 200 according to an embodiment of this application.

As shown in FIG. 2A, the user interface 10 may include a window 101, a curve 102, and a window 103.

The window 101 may be used to display related information of the electronic device 200, for example, a device name, a connection status, remaining initialization time, and remaining available time.

The connection status is a status of the communication connection established between the electronic device 100 and the electronic device 200. If there is a communication connection established between the electronic device 100 and the electronic device 200, the connection status may be "connected". If the communication connection between the electronic device 100 and the electronic device 200 is disconnected, the connection status may be "not connected."

The remaining initialization time is remaining time required by the electronic device 200 to complete initialization. The electronic device 100 needs to output the body indicator data collected by the electronic device 200, namely, the blood glucose data, only after countdown of the remaining initialization time changes to 0. This is because after being worn by the user, the electronic device 200 undergoes an initialization phase in which an output value is unstable for a period of time. Therefore, in embodiments of this application, after being worn by the user, the electronic device 200 may start to record time elapsed since the electronic device 200 is worn by the user, so that the electronic device 100 calculates the remaining initialization time based on the time. In this way, the user can learn about a duration for which the user needs to wait before viewing the blood glucose data collected by the electronic device 200.

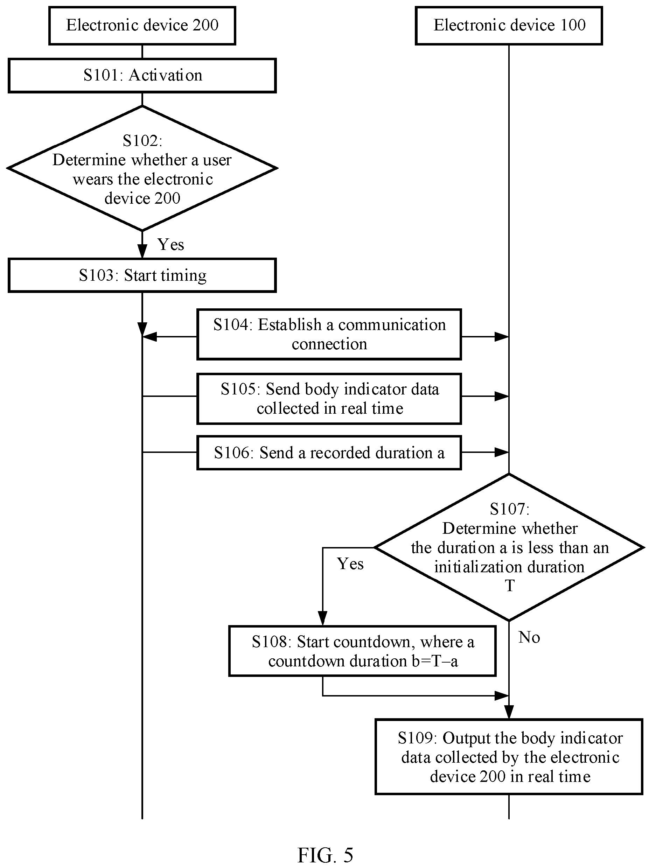

For a specific detailed process in which the electronic device 100 determines the remaining initialization time, refer to subsequent related descriptions in FIG. 5. Details are not described herein.

The remaining available time is remaining time that can be used by the electronic device 200 before the electronic device 200 expires. The electronic device 200 cannot continue to be used due to factors such as component aging and the immune response of the user after the electronic device 200 is worn for a period of time, for example, 15 days. Therefore, when the remaining available time changes to 0 or is about to change to 0, the user may wear a new device configured to detect the body indicator, for example, the electronic device 300, so that the user can continuously monitor the body indicator of the user.

As shown in FIG. 2A, the device name "CGM_1", the connection status "connected", and the remaining initialization time "10 min" of the electronic device 200 may be displayed on the window 101.

The curve 102 may be used to display, after the initialization of the electronic device 200 ends, that is, after the remaining initialization time in the window 101 changes to 0, a blood glucose curve formed by connecting, in a time sequence, the blood glucose data collected by the electronic device 200.

For example, after the remaining initialization time changes to 0, the electronic device 100 may send, to the electronic device 200, indication information for requesting to obtain blood glucose data. In response to the indication information, the electronic device 200 sends, to the electronic device 100, blood glucose data currently collected in real time, so that the electronic device 100 displays a blood glucose curve based on the blood glucose data. Alternatively, after establishing the communication connection to the electronic device 200, the electronic device 100 may continuously obtain blood glucose data collected by the electronic device 200, and after the remaining initialization time changes to 0, display a blood glucose curve based on the blood glucose data collected by the electronic device 200 in real time.

The window 103 may include an implantation reminder switch 103A and a reminder time setting item 103B. The implantation reminder switch 103A may be used to enable or disable an implantation reminder function based on a user operation. As shown in FIG. 2A, the implantation reminder switch 103A is in an enabled state. If the implantation reminder function is enabled on the electronic device 100, when specified time arrives, the electronic device 100 may remind the user to implant a next device configured to monitor blood glucose of the user, for example, the electronic device 300. The reminder time setting item 103B may be used to set time at which the electronic device 100 performs implantation reminder. For example, if the time set by the reminder time setting item 103B is 12 h, when the remaining available time of the electronic device 100 is 12 h, the electronic device 100 may remind the user to implant a next device configured to monitor the blood glucose of the user.

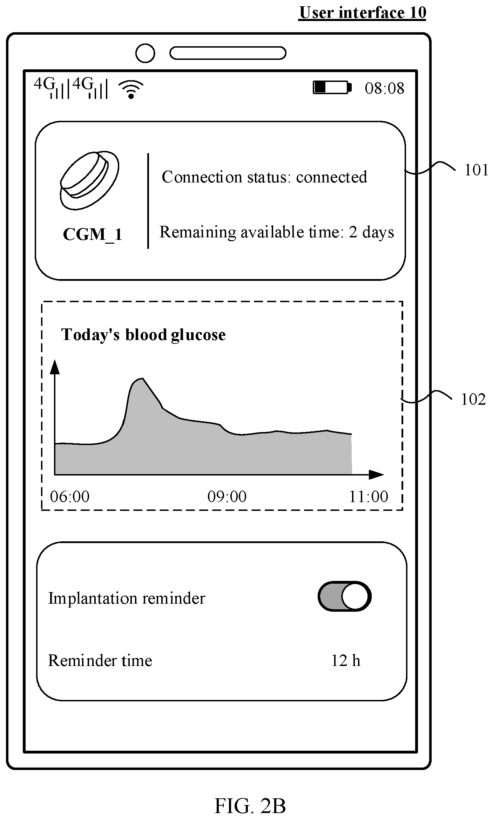

For example, if the electronic device 200 has been initialized, and the remaining available time of the electronic device 200 is two days, the user interface 10 shown in FIG. 2A may be updated to the user interface 10 shown in FIG. 2B.

As shown in FIG. 2B, the remaining available time "2 days" may be displayed on the window 101, and a blood glucose curve 102 may show a blood glucose curve formed by connecting blood glucose data collected by the electronic device 200 in real time.

It may be understood that, in addition to being used to display the blood glucose curve obtained by connecting the blood glucose data collected by the electronic device 200, the user interface 10 may be used to display one or more blood glucose values collected by the electronic device 200. A manner in which the electronic device 100 displays the blood glucose data collected by the electronic device 200 is not limited in embodiments of this application.

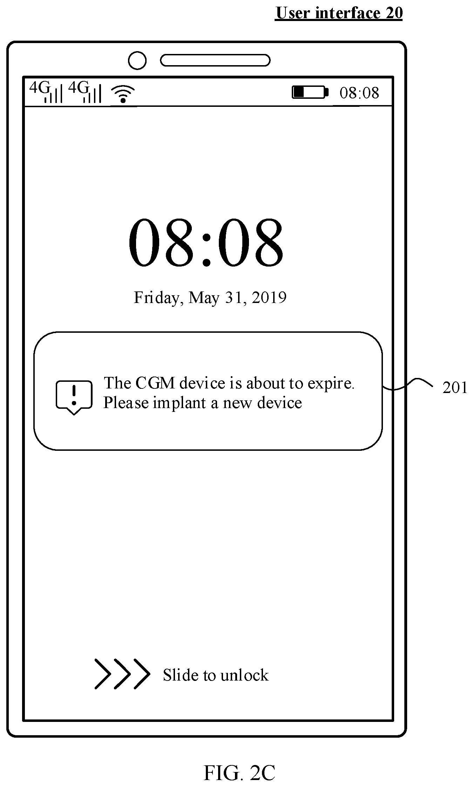

For example, if the time set by the electronic device 100 through the reminder time setting item 103B in the user interface 10 is 12 h, the electronic device 100 may display a user interface 20 shown in FIG. 2C when the remaining available time of the electronic device 200 is 12 h.

As shown in FIG. 2C, the user interface 20 may include prompt information 201. The prompt information 201 may be used to remind the user to implant a new device. For example, the prompt information 201 may be displayed as a text "The CGM device is about to expire. Please implant a new device".

It may be understood that FIG. 2C shows that the electronic device 100 displays, on a lock screen, the prompt information used to remind the user to implant the new device. In another embodiment of this application, the electronic device 100 may further display the prompt information on another user interface. This may be specifically determined by a user interface displayed by the electronic device 100 when reminder time arrives. This is not limited in embodiments of this application.

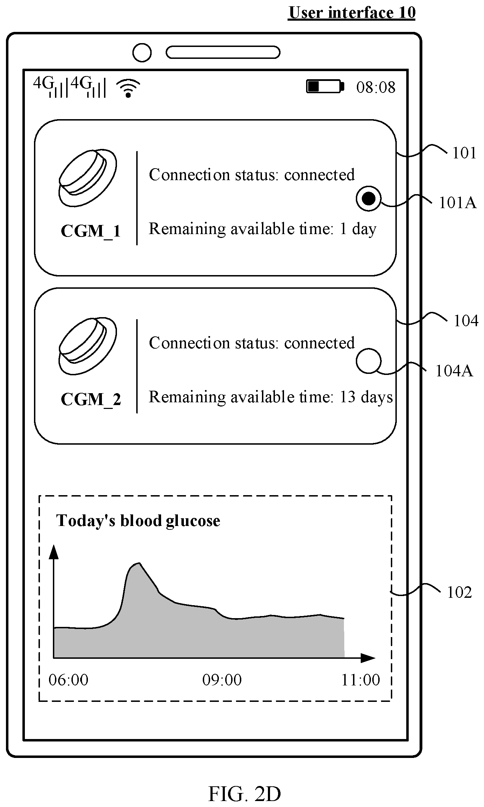

Further, if the user implants the electronic device 300 before the electronic device 200 expires, and the electronic device 100 establishes the communication connection to the electronic device 300, the user interface 10 may be updated to the user interface 10 shown in FIG. 2D.

As shown in FIG. 2D, the user interface 10 may include the window 101, a window 104, and the curve 102.

The window 101 may be used to display the related information of the electronic device 200, for example, the device name, the connection status, the remaining initialization time, and the remaining available time. As shown in FIG. 2D, the device name "CGM_1", the connection status "connected", and the remaining available time "1 day" of the electronic device 200 may be displayed on the window 101.

The window 104 may be used to display related information of the electronic device 300, for example, a device name, a connection status, remaining initialization time, and remaining available time. As shown in FIG. 2D, the device name "CGM_2", the connection status "connected", and the remaining available time "13 days" of the electronic device 300 may be displayed on the window 104.

In addition, the window 101 may further include an icon 101A, and the window 104 may further include an icon 104A. The icon 101A and the icon 104A each may include two states: a selected state and an unselected state. The electronic device 100 may change a state of the icon 101A or the icon 104A based on a user operation performed by the user on the icon 101A or the icon 104A, for example, a tap operation. As shown in FIG. 2D, the icon 101A may be currently considered to be in the selected state, and the icon 104A may be considered to be in the unselected state. The electronic device 100 may display the blood glucose data of the device in the selected state in the user interface 10.

As shown in FIG. 2D, the curve 102 is used to display a blood glucose curve drawn based on the blood glucose data collected by the electronic device 200 in the selected state.

It may be understood that, if the icon 101A shown in FIG. 2D is in the unselected state, and the icon 104A is in the selected state, the curve 102 may be used to display a blood glucose curve formed by connecting blood glucose data collected by the electronic device 300. Alternatively, if both the icon 101A and the icon 104A shown in FIG. 2D are in the selected state, the curve 102 may be used to display the blood glucose curve formed by connecting the blood glucose data collected by the electronic device 200 and the blood glucose curve formed by connecting the blood glucose data collected by the electronic device 300.

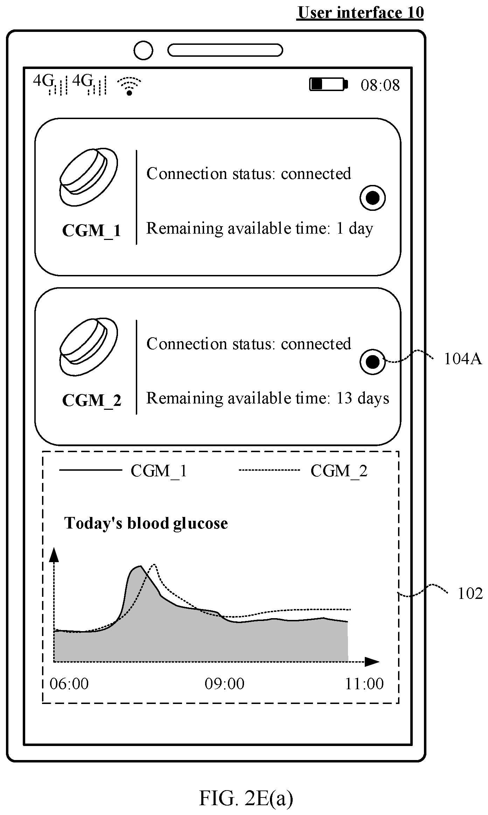

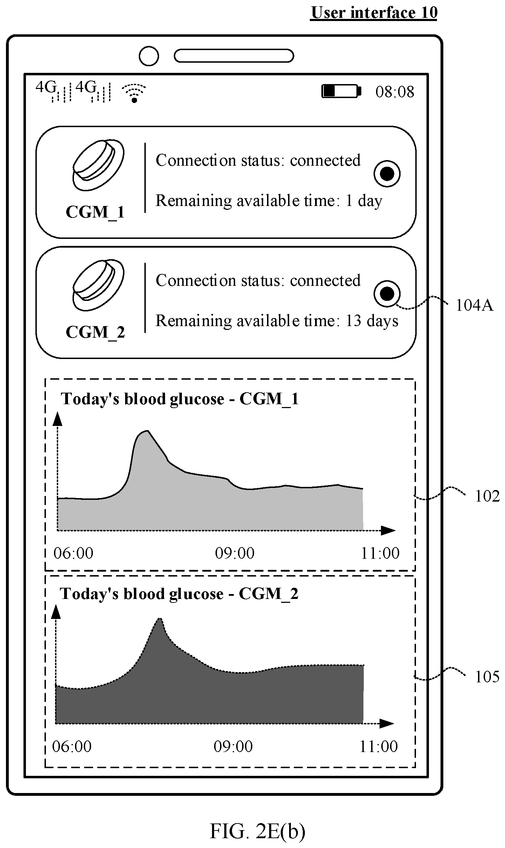

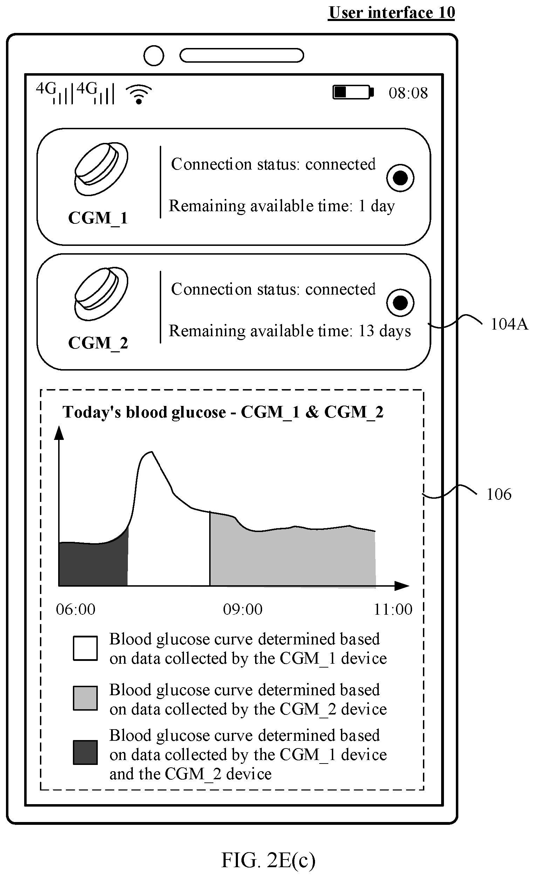

If the electronic device 100 detects a user operation performed on the icon 104A shown in FIG. 2D, and changes a status of the icon 104A, to make the icon 104A be in the selected state, the electronic device 100 may display the user interface 10 shown in FIG. 2E(a), FIG. 2E(b), or FIG. 2E(c).

As shown in FIG. 2E(a), in comparison with FIG. 2D, the icon 104A is in the selected state, and the blood glucose curve formed by connecting the blood glucose data collected by the electronic device 200 and the blood glucose curve formed by connecting the blood glucose data collected by the electronic device 300 are simultaneously displayed in the curve 102, and the two blood glucose curves are displayed in a same coordinate system.

In this way, the user may view, in the same coordinate system, blood glucose conditions monitored by a plurality of devices worn by the user. Optionally, a plurality of blood glucose curves corresponding to the plurality of devices may be respectively displayed with different display effects.

As shown in FIG. 2E(b), in comparison with FIG. 2D, the icon 104A is in the selected state, and a curve 105 is newly displayed in the user interface 10. The curve 102 in the user interface 10 is used to display the blood glucose curve drawn based on the blood glucose data collected by the electronic device 200, and the curve 105 in the user interface 10 is used to display the blood glucose curve formed by connecting the blood glucose data collected by the electronic device 300.

In this way, the user may view, respectively in different coordinate systems, blood glucose conditions monitored by different devices worn by the user.

It should be noted that, when the user wears a plurality of CGM devices at the same time, different CGM devices are worn on different parts. Even if data collected by these CGM devices is accurate, blood glucose data actually collected by the different CGM devices may be different due to different blood glucose contents of the different parts of the user.

As shown in FIG. 2E(c), in comparison with FIG. 2D, the icon 104A is in the selected state, and the curve 102 is updated to a curve 106. The curve 106 may be used to display a blood glucose curve drawn by the electronic device 100 based on the blood glucose data collected by the electronic device 200 and the blood glucose data collected by the electronic device 300.

For example, the blood glucose curve may include any one or more of the following: a blood glucose curve segment drawn based on the blood glucose data collected by the electronic device 200, a blood glucose curve segment drawn based on the blood glucose data collected by the electronic device 300, and a blood glucose curve segment drawn based on blood glucose data obtained through calculation on the blood glucose data collected by the electronic device 200 and the blood glucose data collected by the electronic device 300, for example, blood glucose data obtained by averaging blood glucose values collected by the electronic device 200 and the electronic device 300 at the same time.

In this way, the user may view a blood glucose condition of the user that is obtained by combining blood glucose data monitored by the plurality of devices.

Whether the blood glucose curve displayed in the curve 106 is drawn based on blood glucose data collected by one of the devices or drawn based on blood glucose data collected by two devices may be determined based on accuracy of blood glucose data collected by the device. For example, if the electronic device 100 identifies that blood glucose data collected by the electronic device 200 in a first time period is accurate, and blood glucose data collected by the electronic device 300 in the first time period is inaccurate, a curve segment in the first time period in the blood glucose curve drawn by the electronic device 100 is drawn based on the blood glucose data collected by the electronic device 200 in the first time period. For another example, if the electronic device 100 identifies that both the blood glucose data collected by the electronic device 200 and the blood glucose data collected by the electronic device 300 in a second time period are accurate, a curve segment in the second time period in the blood glucose curve drawn by the electronic device 100 is obtained based on the blood glucose data collected by the electronic device 200 or the blood glucose data collected by the electronic device 300 in the second time period, or the curve segment in the second time period is obtained based on the blood glucose data collected by the electronic device 200 in the second time period and the blood glucose data collected by the electronic device 300 in the second time period, for example, drawn based on an average value of the blood glucose data collected by the electronic device 200 in the second time period and the blood glucose data collected by the electronic device 300 in the second time period.

It may be understood that, it is considered that spliced blood glucose curve segments drawn based on different data may be discontinuous due to discontinuous data. Therefore, the electronic device 100 may perform smoothing on a connection part between the curve segments, to ensure that the blood glucose curve segments drawn based on the different data can be spliced into a continuous curve. A manner of curve smoothing is not limited in embodiments of this application.

It can be learned from FIG. 2A to FIG. 2E(c) that, when the electronic device 100 has established a communication connection to one CGM device, the electronic device 100 may remind, before the CGM device expires, the user in time to wear a new CGM device, to ensure that the electronic device 100 can continuously display a blood glucose condition of the user to the user. In addition, when the user simultaneously wears a plurality of CGM devices, the electronic device 100 may simultaneously display blood glucose data collected by the plurality of CGM devices, so that the user can learn about the blood glucose condition of the user from a plurality of aspects.

FIG. 3A to FIG. 3F show related user interfaces for the electronic device 100 to identify accuracy of data collected by the electronic device 200 or the electronic device 300.

When the electronic device 100 simultaneously obtains the blood glucose data collected by the electronic device 200 and the blood glucose data collected by the electronic device 300, the electronic device 100 may identify accuracy of the blood glucose data based on the blood glucose data collected by the electronic device 200 and the blood glucose data collected by the electronic device 300.

In a scenario, early sensitivity attenuation (early sensitivity attenuation, ESA) of the CGM device causes an inaccurate output value of the CGM device. Specifically, in an early wearing stage of the CGM device, for example, in the first 12 to 24 hours after the CGM device starts to be worn, after a probe of the CGM device is implanted under the skin of the user, phenomena such as bleeding caused by trauma and an immune response of the body may easily occur. These phenomena may cause temporary attenuation of sensitivity of a sensor of the CGM device, and consequently, a blood glucose value collected by the CGM device decreases sharply.

The CGM device recovers by itself after early sensitivity attenuation. Therefore, after the early sensitivity attenuation occurs on the CGM device, blood glucose data collected by the CGM device shows a change trend of first rapidly decreasing and then rapidly increasing.

The electronic device 100 may identify, based on the blood glucose change trend, whether the CGM device is in an early sensitivity attenuation phenomenon. In addition, due to a physical reason of the user, blood glucose of the user may also have a change trend of first rapidly decreasing and then rapidly increasing. Therefore, to prevent the electronic device 100 from incorrectly identifying a blood glucose fluctuation caused by the physical reason of the user as early sensitivity attenuation of the CGM, if the user wears a plurality of CGM devices, blood glucose data collected by the plurality of CGM devices may be used to determine whether blood glucose data is abnormal due to early sensitivity attenuation of a CGM device, so as to identify accuracy of the blood glucose data collected by the CGM device.

In other words, for CGM devices worn by the user at different time, the electronic device 100 may identify, based on blood glucose data of a CGM device worn first, accuracy of blood glucose data of a CGM device worn later by the user.

For example, it is assumed that the electronic device 200 is a CGM device worn by the user first, and the electronic device 300 is a CGM device worn by the user later. The electronic device 100 may determine, based on the blood glucose data collected by the electronic device 200, whether early sensitivity attenuation occurs on the electronic device 300 in an early wearing stage, to identify accuracy of the blood glucose data collected by the electronic device 300.

In embodiments of this application, the early sensitivity attenuation may also be referred to as early attenuation, early sensitivity reduction, or the like. The name is not limited in embodiments of this application.

If the electronic device 100 identifies, based on the data collected by the electronic device 200, that a blood glucose value collected by the electronic device 300 is inaccurate, the electronic device 100 may display the user interface 10 shown in FIG. 3A.

As shown in FIG. 3A, the user interface 10 may include prompt information 107. The prompt information 107 may be used to give the user a prompt that the blood glucose value currently collected by the electronic device 300 is abnormal. The prompt information 107 may include a reject option 107A and an OK option 107B. The reject option 107A may be used to trigger the electronic device 100 to reject using the abnormal blood glucose value collected by the electronic device 300 as an actual blood glucose value of the user. In other words, if the electronic device 100 detects a user operation performed by the user on the reject option 107A, it indicates that the user considers that the abnormal blood glucose value collected by the electronic device 300 is inaccurate and cannot reflect an actual physical condition of the user. The OK option 107B may be used to trigger the electronic device 100 to agree to use the abnormal blood glucose value collected by the electronic device 300 as the actual blood glucose value of the user. In other words, if the electronic device 100 detects a user operation performed by the user on the OK option 107B, it indicates that the user considers that the abnormal blood glucose value collected by the electronic device 300 is accurate and reflects an actual blood glucose condition of the user.

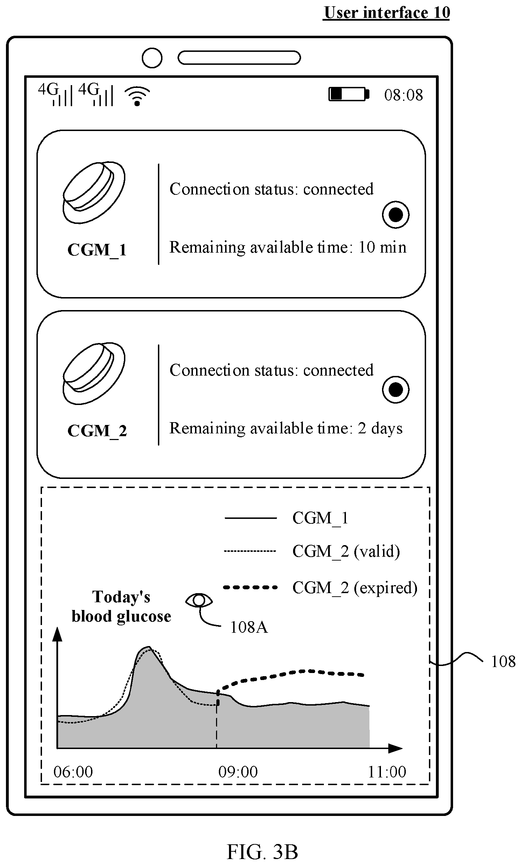

If the electronic device 100 detects the user operation performed by the user on the reject option 107A, for example, a tap operation, it indicates that the user considers that the abnormal blood glucose value collected by the electronic device 300 is inaccurate. Therefore, when displaying the blood glucose data collected by the electronic device 300, the electronic device 100 may display the abnormal blood glucose value in a differentiated manner, to indicate that the abnormal blood glucose value cannot be used to reflect the actual blood glucose condition of the user. For example, the electronic device 100 may display the user interface 10 shown in FIG. 3B.

As shown in FIG. 3B, the user interface 10 may include a curve 108. The curve 108 may be used to display, in a same coordinate system, blood glucose curves respectively formed by connecting the blood glucose data collected by the electronic device 200 and the blood glucose data collected by the electronic device 300. If the electronic device 100 determines that a blood glucose value collected by the electronic device 300 in a time period of 09:00 to 11:00 is inaccurate, in a blood glucose curve corresponding to the electronic device 300 displayed in the curve 108, a blood glucose curve corresponding to 09:00 to 11:00 may be displayed in bold, to distinguish the blood glucose curve from blood glucose curves in other time periods.

Optionally, the curve 108 may include a hide icon 108A, and the hide icon 108A may be used to trigger to hide a curve segment that is drawn based on inaccurate data and that is in a curve displayed in the curve 108. In another implementation, the hide icon 108A may be used to trigger to hide a curve segment that is drawn based on accurate data and that is in the curve displayed in the curve 108.

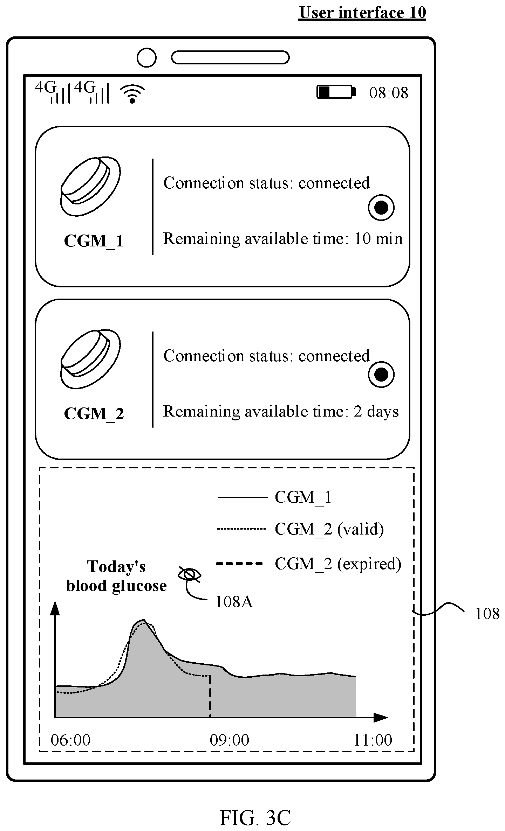

For example, as shown in FIG. 3B, if the electronic device 100 detects a user operation performed by the user on the hide icon 108A, for example, a tap operation, the electronic device 100 may hide the curve segment that is drawn based on the inaccurate data and that is in the curve displayed in the curve 108, that is, the electronic device 100 may update the curve displayed in the curve 108 to a curve displayed in the curve 108 shown in FIG. 3C.

As shown in FIG. 3C, the curve displayed in the curve 108 does not include a curve segment drawn based on data collected by the CGM_2 in the time period of 09:00 to 11:00.

Further, after some curve segments in the curve 108 are hidden, the hide icon 108A shown in FIG. 3B may be updated to the hide icon 108A shown in FIG. 3C. In addition, the electronic device 100 may further detect again a user operation performed on the hide icon 108A shown in FIG. 3C, for example, a tap operation, to cancel hiding of a hidden curve segment in the curve 108.

In another scenario, late sensor attenuation (late sensor attenuation, LSA) of a CGM device also causes an inaccurate output value of the CGM device. Specifically, in a late wearing stage of the CGM device, for example, 12 to 24 hours before the CGM device expires, the CGM device may expire in advance due to a factor like a part of a wearing component of the CGM device falling off due to excessively long wearing time or an immune response of the user, resulting in a sharp decrease in a blood glucose value collected by the CGM device.

The CGM device cannot recover by itself after the late sensor attenuation. Therefore, after the late sensor attenuation on the CGM device, the blood glucose data collected by the CGM device shows a continuous decreasing change trend.

The electronic device 100 may identify, based on the blood glucose change trend, whether the CGM device is in a late sensor attenuation phenomenon. In addition, due to the physical reason of the user, the blood glucose of the user may also have a rapidly decreasing change trend. Therefore, to prevent the electronic device 100 from incorrectly identifying a blood glucose fluctuation caused by the reason of the user as late sensor attenuation of the CGM device, if the user wears a plurality of CGM devices, blood glucose data collected by the plurality of CGM devices may be used to determine whether blood glucose data is abnormal due to late sensor attenuation of a CGM device, so as to identify accuracy of the blood glucose data collected by the CGM device.

In other words, for CGM devices worn by the user at different time, the electronic device 100 may identify, based on blood glucose data of a CGM device worn later, accuracy of blood glucose data of a CGM device worn first by the user.

For example, it is assumed that the electronic device 200 is a CGM device worn by the user first, and the electronic device 300 is a CGM device worn by the user later. The electronic device 100 may determine, based on the blood glucose data collected by the electronic device 300, whether late sensor attenuation occurs on the electronic device 200 in a late wearing stage, to identify accuracy of the blood glucose data collected by the electronic device 200.

In embodiments of this application, the late sensor attenuation may also be referred to as sensor life termination, late attenuation, or the like. The name is not limited in embodiments of this application.

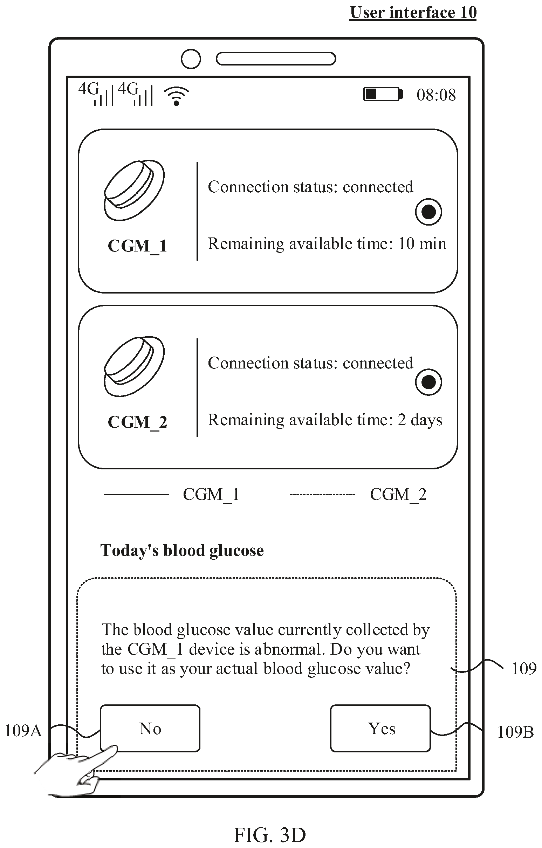

If the electronic device 100 identifies, based on the data collected by the electronic device 300, that a blood glucose value collected by the electronic device 200 is inaccurate, the electronic device 100 may display the user interface 10 shown in FIG. 3D.

As shown in FIG. 3D, the user interface 10 may include prompt information 109. The prompt information 109 may be used to give the user a prompt that the blood glucose value currently collected by the electronic device 200 is abnormal. The prompt information 109 may include a reject option 109A and an OK option 109B. The reject option 109A may be used to trigger the electronic device 100 to reject using the abnormal blood glucose value collected by the electronic device 200 as an actual blood glucose value of the user. In other words, if the electronic device 100 detects a user operation performed by the user on the reject option 109A, it indicates that the user considers that the abnormal blood glucose value collected by the electronic device 200 is inaccurate and cannot reflect an actual blood glucose condition of the user. The OK option 109B may be used to trigger the electronic device 100 to agree to use the abnormal blood glucose value collected by the electronic device 200 as the actual blood glucose value of the user. In other words, if the electronic device 100 detects a user operation performed by the user on the OK option 109B, it indicates that the user considers that the abnormal blood glucose value collected by the electronic device 200 is accurate and reflects an actual blood glucose condition of the user.

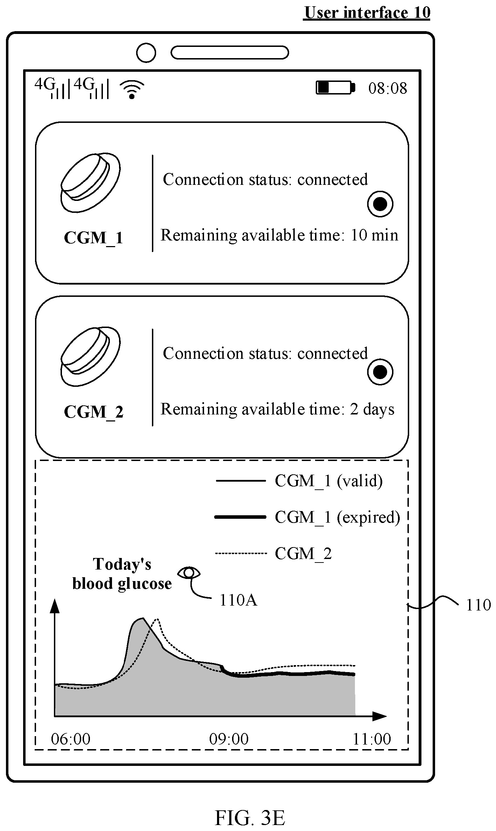

If the electronic device 100 detects the user operation performed by the user on the reject option 109A, for example, a tap operation, it indicates that the user considers that the abnormal blood glucose value collected by the electronic device 200 is inaccurate. Therefore, when displaying the blood glucose data collected by the electronic device 200, the electronic device 100 may display the abnormal blood glucose value in a differentiated manner, to indicate that the abnormal blood glucose value cannot be used to reflect the actual blood glucose condition of the user. For example, the electronic device 100 may display the user interface 10 shown in FIG. 3E.

As shown in FIG. 3E, the user interface 10 may include a curve 110. The curve 110 may be used to display, in a same coordinate system, blood glucose curves respectively formed by connecting the blood glucose data collected by the electronic device 200 and the blood glucose data collected by the electronic device 300. If the electronic device 100 determines that a blood glucose value collected by the electronic device 200 in the time period of 09:00 to 11:00 is inaccurate, in a blood glucose curve corresponding to the electronic device 200 displayed in the curve 110, a blood glucose curve corresponding to 09:00 to 11:00 may be displayed in bold, to distinguish the blood glucose curve from blood glucose curves in other time periods.

It may be understood that, in addition to highlighting a curve segment drawn based on inaccurate data, the electronic device 100 may highlight a curve segment drawn based on accurate data. In other words, the curve segment drawn based on the accurate data and the curve segment drawn based on the inaccurate data may have different display effects, and the display effects may include a color, a line thickness, a line type, and the like. The user may distinguish data with different accuracy based on different display effects.

In addition, the electronic device 100 may display the curve segment drawn based on the accurate data, and does not display the curve segment drawn based on the inaccurate data; or the electronic device 100 may display the curve segment drawn based on the inaccurate data, and does not display the curve segment drawn based on the accurate data. Further, the electronic device 100 may select, based on a user operation, a curve segment to be displayed. For example, when displaying a curve segment drawn based on the accurate data collected by the electronic device 200, the electronic device 100 may detect a user operation, and newly display a curve segment drawn based on the inaccurate data collected by the electronic device 200. In this way, the user may control data displayed by the electronic device 100, and filter, based on data accuracy, the data displayed by the electronic device 100.

Optionally, the curve 110 may include a hide icon 110A, and the hide icon 110A may be used to trigger to hide a curve segment that is drawn based on inaccurate data and that is in a curve displayed in the curve 110. In another implementation, the hide icon 110A may be used to trigger to hide a curve segment that is drawn based on accurate data and that is in the curve displayed in the curve 108.

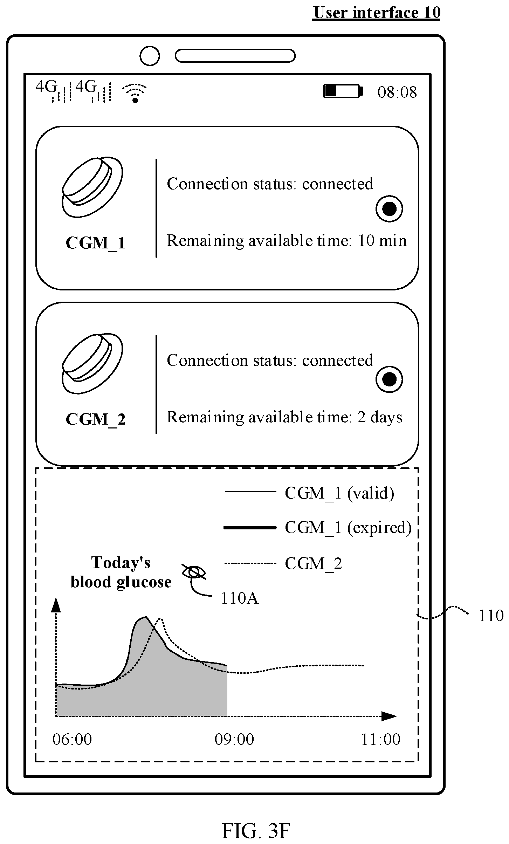

For example, as shown in FIG. 3E, if the electronic device 100 detects a user operation performed by the user on the hide icon 110A, for example, a tap operation, the electronic device 100 may hide the curve segment that is drawn based on the inaccurate data and that is in the curve displayed in the curve 110, that is, the electronic device 100 may update the curve displayed in the curve 110 to a curve displayed in the curve 110 shown in FIG. 3F.

As shown in FIG. 3F, the curve displayed in the curve 110 does not include a curve segment drawn based on data collected by the CGM_1 in the time period of 09:00 to 11:00.

Further, after some curve segments in the curve 110 are hidden, the hide icon 110A shown in FIG. 3E may be updated to the hide icon 110A shown in FIG. 3F. In addition, the electronic device 100 may further detect a user operation performed on the hide icon 110A shown in FIG. 3F, for example, a tap operation, to cancel hiding of a hidden curve segment in the curve 110.

It can be learned from FIG. 3A to FIG. 3F that, when the electronic device 100 may obtain blood glucose data collected by a plurality of CGM devices, the electronic device 100 may identify, based on the blood glucose data collected by the plurality of CGM devices, accuracy of blood glucose data collected by one of the CGM devices, to ensure that the electronic device 100 can display an actual blood glucose condition of the user, and provide an accurate blood glucose evaluation result for the user.

It should be noted that the user interfaces shown in FIG. 2A to FIG. 2E(c) and FIG. 3A to FIG. 3F are merely examples for description, and do not constitute a limitation on embodiments of this application.

Although the electronic device 200 or the electronic device 300 may start to measure the body indicator of the user after the electronic device 200 or the electronic device 300 is worn, the electronic device 200 or the electronic device 300 usually needs to wait for a period of time to collect actual and accurate body indicator data of the user.

A CGM device is used as an example. Because after a probe of the CGM device is implanted under skin, an electrochemical reaction needs to be generated between the probe and glucose in tissue fluid, and a current formed by electronic directional movement is used to reflect a blood glucose value of the user, after the CGM device is worn, the user needs to wait for a period of time, for example, half an hour or one hour, for completing device initialization, and then, the CGM device can collect a stable and accurate blood glucose value.

In embodiments of this application, a duration for the device configured to monitor the body indicator, for example, the CGM device, to complete initialization is referred to as an initialization duration.

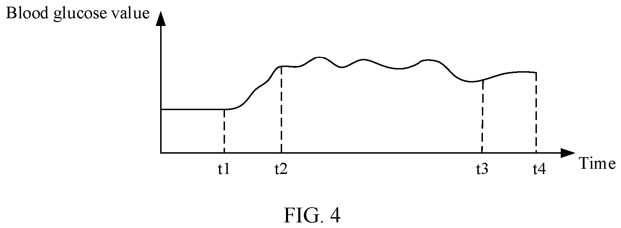

For example, FIG. 4 shows, by using an example in which the electronic device 200 is a CGM device, a blood glucose change curve collected by the electronic device 200 after the electronic device 200 is worn by the user.

As shown in FIG. 4, t1 is a time point at which the user wears the electronic device 200, t1-t2 is a time period in which the electronic device 200 performs initialization, t2 is a time point at which the electronic device 200 completes the initialization, and after t2, the electronic device 200 may normally collect a blood glucose value.

It can be learned that, after the user wears the electronic device 200, the electronic device 200 needs to perform initialization for a period of time, and a blood glucose value collected in an initialization stage cannot be used to reflect an actual blood glucose value of the user. Only after the initialization ends, the blood glucose value collected by the electronic device 200 can represent an actual blood glucose value of the user.

Further, because the CGM device expires after being worn for a period of time, for example, 15 days, the user needs to replace the CGM device with a new CGM device to continue to monitor the body indicator. As shown in FIG. 4, t4 is a time point at which the electronic device 200 expires.

In addition, the electronic device 100 may remind the user to replace the CGM device in time before the electronic device 200 expires. As shown in FIG. 4, t3 is a time point at which the user is reminded that the electronic device 200 is about to expire. An interval duration of t3-t4 may be greater than or equal to the initialization duration of the CGM device, namely, the interval duration of t1-t2. In this way, after the user wears the new CGM device (for example, the electronic device 300) and the new CGM device completes initialization and can output an actual and stable blood glucose value, the electronic device 200 can expire, to ensure that the user can continuously monitor the blood glucose condition of the user.

To ensure that the electronic device 100 can output stable and actual body indicator data after the user wears the electronic device 200, a general practice is as follows: After the electronic device 100 establishes the communication connection to the electronic device 200, the electronic device 100 counts down the initialization duration, and then outputs body indicator data collected by the electronic device 200 in real time. However, it can be learned from FIG. 4 that the initialization starts when the electronic device 200 is worn by the user. If the countdown starts from the establishment of the communication connection, it is equivalent to that the user waits for an extra period of time, and consequently, a waiting duration of the user is inappropriate.

Therefore, an embodiment of this application provides an initialization method, so that the electronic device 100 can output body indicator data after the electronic device 200 completes initialization, to shorten waiting time of the user.

**FIG. 5** **is a schematic flowchart of an initialization method according to an embodiment of this application.**

**S101:** The electronic device 200 is activated.

Activating the electronic device 200 may mean using a power supply in the electronic device 200 to supply power to hardware in the electronic device 200, for example, a micro controller unit (micro controller unit, MCU) and a sensor, so that the electronic device 200 can start to operate and monitor the body indicator of the user.

For example, the electronic device 200 may be activated before the user wears the electronic device 200. In this way, after the electronic device 200 is activated, a time point at which the user wears the electronic device 200 may be identified based on data collected by the sensor, and timing starts when the user wears the electronic device 200, that is, step S 102 to step S104 are performed.

It may be understood that, if the electronic device 200 is activated when the user wears the electronic device 200, the electronic device 200 may start timing after the electronic device 200 is activated, that is, step S104 is performed. In this case, step S102 and step S103 are optional steps.

The electronic device 200 may be activated through a user operation performed by the user on an activation switch of the electronic device 200. Alternatively, the electronic device 200 may be automatically activated through cooperation between components of the electronic device 200. For example, the electronic device 200 may sense a change of an ambient environment by using an optical sensor, to automatically activate the electronic device 200. How the electronic device 200 is activated is not limited in embodiments of this application.

**S102:** The electronic device 200 determines whether the user wears the electronic device 200.

For example, the electronic device 200 may determine, based on data collected by the sensor, whether the user wears the electronic device 200. If the data collected by the sensor meets a first preset condition, the electronic device 200 determines that the user wears the electronic device 200. If the data collected by the sensor does not meet the first preset condition, the electronic device 200 determines that the user does not wear the electronic device 200.

The sensor may be a sensor configured to monitor the body indicator of the user, and the electronic device 100 may determine the body indicator of the user based on the data collected by the sensor.

For example, the electronic device 200 is a CGM device. The sensor may include an electrochemical sensor, a temperature sensor, and the like. When the user wears the electronic device 200, the electrochemical sensor is implanted under skin of the user. The electrochemical sensor may be configured to detect a current value under the skin, so that the electronic device 200 obtains a blood glucose value through calculation based on the current value. The temperature sensor may be configured to detect a temperature of the ambient environment, and the electronic device 200 may correct, based on the temperature, the blood glucose value obtained by the electronic device 100 through calculation, so that the blood glucose value obtained by the electronic device 100 through calculation is more accurate. For example, the first preset condition may be that the current value collected by the electrochemical sensor changes, and a temperature value collected by the temperature sensor changes, or a change amount of the current value collected by the electrochemical sensor is greater than a threshold, and a change amount of a temperature value collected by the temperature sensor is greater than a threshold. The change of the current value collected by the electrochemical sensor indicates that the electrochemical sensor may be implanted under the skin of the user. That the temperature value collected by the temperature sensor changes indicates that the temperature sensor is close to the skin of the user, and the electronic device 200 may be attached to the skin of the user. Therefore, the change of the value collected by the electrochemical sensor and the change of the value collected by the temperature sensor indicate that the electronic device 200 is successfully worn.

In some implementations, if the data collected by the sensor meets a second preset condition, it is determined that the user fails to wear the electronic device 200. In this case, the electronic device 200 or the electronic device 100 may output prompt information, to give the user a prompt that the electronic device 200 fails to be worn, and to prompt the user to check a wearing status of the electronic device 200. For example, the sensor includes an electrochemical sensor and a temperature sensor. The second preset condition may indicate that the current value collected by the electrochemical sensor is 0, and the temperature value collected by the temperature sensor changes, for example, the value of the temperature changes to a temperature close to a human skin temperature (for example, 30°C). That the current value collected by the electrochemical sensor is 0 indicates that the electrochemical sensor may not be implanted under the skin of the user. That the temperature value collected by the temperature sensor changes indicates that the temperature sensor is close to the skin of the user, and the electronic device 200 may be attached to the skin of the user. Therefore, it can be learned from data collected by the electrochemical sensor and the temperature sensor that the electronic device 200 fails to be worn.

If the electronic device 200 determines that the user wears the electronic device 200, step S103 is performed. Otherwise, the electronic device 200 may continuously perform step S102 to determine whether the user wears the electronic device 200.

It may be understood that, in step S102, determining whether the user wears the electronic device 200 may alternatively be performed by the electronic device 100. In this case, the electronic device 200 only needs to collect the data collected by the sensor, and after establishing a communication connection to the electronic device 100, sends, to the electronic device 100, the data collected by the sensor in the electronic device 200, and the electronic device 100 determines whether the user wears the electronic device 200. In this way, a calculation amount of the electronic device 200 can be reduced.

**S103:** The electronic device 200 starts timing.

After determining that the user wears the electronic device 200, the electronic device 200 may start timing and record a duration elapsed since the user starts to wear the electronic device 200.

In a specific implementation, a timing module, for example, a real time clock (real time clock, RTC) chip, may be configured in the electronic device 200, and timing is performed through the timing module.

It may be understood that the electronic device 200 may alternatively not need to perform step S103 after determining that the user wears the electronic device 200, but perform timing in a time sequence of the data collected by the sensor. In this way, after establishing the communication connection to the electronic device 100, the electronic device 200 sends, to the electronic device 100 at a time node of each data collection, the data collected by the sensor, and the electronic device 100 may backtrack and identify a time point at which the data collected by the sensor meets the first preset condition, to obtain the duration elapsed since the user starts to wear the electronic device 200.

**S104:** The electronic device 200 establishes the communication connection to the electronic device 100.

For example, after being activated, the electronic device 200 may send broadcast information, for example, a Bluetooth low energy (Bluetooth low energy, BLE) broadcast signal, to the ambient environment. After the electronic device 100 obtains the broadcast information and sends, to the electronic device 200, a message indicating that the electronic device 100 agrees to establish the connection, the electronic device 200 and the electronic device 100 can complete the connection establishment.

In some implementations, after obtaining the broadcast signal sent by the electronic device 200, the electronic device 100 may display prompt information, for the user to choose whether to agree to establish the communication connection to the electronic device 200. In addition, after detecting an operation that the user agrees to establish the communication connection, the electronic device 100 sends, to the electronic device 200, the message indicating that the electronic device 100 agrees to establish the communication connection.

It may be understood that step S104 may be performed after any one of the foregoing steps. An execution sequence of step S104 is not limited in embodiments of this application.

**S105:** The electronic device 200 sends, to the electronic device 100, body indicator data collected in real time.

After the electronic device 200 is worn by the user, the electronic device 200 may start to measure the body indicator of the user, and collect the body indicator data of the user.

For example, the electronic device 200 may send, to the electronic device 100 in any one of the following cases, the body indicator data collected in real time:
(1) The electronic device 200 may send, to the electronic device 100 after establishing the communication connection to the electronic device 100, the body indicator data collected in real time.
(2) The electronic device 200 sends, to the electronic device 100 after receiving a request for monitoring the body indicator from the electronic device 100, the body indicator data collected in real time.
(3) The electronic device 200 sends, to the electronic device 100 after the duration elapsed since the electronic device 200 starts to be worn reaches an initialization duration T, the body indicator data collected in real time.

The initialization duration T is a duration elapsed since the electronic device 200 starts to be worn until stable data that can reflect an actual body indicator of the user is collected. The duration may be determined based on a structure and a component of the electronic device 200 and the body indicator collected by the electronic device 200. Different electronic devices may require different initialization durations, and the initialization duration may be half an hour, one hour, or the like.

It may be understood that step S105 may be performed before or after any one of step S104 to step S108. This is not limited in embodiments of this application.

**S106:** The electronic device 200 sends the recorded duration a to the electronic device 100.

After establishing the communication connection to the electronic device 100, the electronic device 200 may send, to the electronic device 100, the duration a recorded from the time when the user starts to wear the electronic device 200. The duration a is used to record the duration elapsed since the electronic device 200 starts to be worn.

For example, after establishing the communication connection to the electronic device 200, the electronic device 100 may send, to the electronic device 200, a request for obtaining the timing duration. After obtaining the request, the electronic device 200 sends the recorded duration a to the electronic device 100. The electronic device 100 may send the request for the timing duration to the electronic device 200 when countdown needs to be performed.

It may be understood that a sequence of step S105 and step S106 does not limit a sequence of performing step S105 and step S106. For example, the electronic device 100 may first perform step S105 and then perform step S106, or first perform step S106 and then perform step S105, or simultaneously perform step S105 and step S106.

**S107:** The electronic device 100 determines whether the duration a is less than the initialization duration T.

If the duration a is greater than or equal to the initialization duration T, it indicates that a duration elapsed since the user wears the electronic device 200 until the electronic device 100 needs to perform countdown is greater than or equal to the initialization duration T. In this case, the body indicator data collected by the electronic device 200 is stable, and can be used to reflect an actual physical condition of the user. Therefore, the electronic device 100 may perform step S109 to directly output the body indicator data collected by the electronic device 200. The data may be the body indicator data collected by the electronic device 200 in real time, or may be body indicator data collected by the electronic device 200 after a duration in which timing starts after the user wears the electronic device 200 reaches T.

If the duration a is less than the initialization duration T, it indicates that the duration elapsed since the user wears the electronic device 200 until the electronic device 100 needs to perform countdown is still less than the initialization duration. In this case, the electronic device 200 is still in an initialization stage, and collected body indicator data is unstable, and cannot be used to reflect the actual physical condition of the user. Therefore, the electronic device 100 may perform step S108 to perform countdown and wait for the electronic device 200 to collect stable body indicator data.

In addition, after the electronic device 200 is activated, the user may forget to wear the electronic device 200, or the user wears the electronic device 200 after long time. The electronic device 200 that is successfully worn only after the sensor is implanted under the skin of the user, for example, the CGM device, needs to be kept in an aseptic state before being implanted under the skin. In this case, if the user forgets to wear the electronic device 200, or an interval between the activation and wearing of the user is long, a risk of infection of the electronic device 200 is increased. As a result, after the electronic device 200 is implanted under the skin of the user, a risk of infection of the user is increased, and physical health of the user is affected.

Therefore, after being activated, the electronic device 200 may record a duration c elapsed from the activation of the electronic device 200 to wearing of the electronic device 200, and remind the user to wear the electronic device 200 in time by using the duration c, or remind the user to cancel wearing when the duration c exceeds a threshold.

Specifically, the electronic device 200 may start timing after step S101, and record a duration elapsed since the activation of the electronic device 200.

In some implementations, if the electronic device 200 identifies that the duration elapsed since the activation is greater than a threshold, for example, 10 minutes, and the user still does not wear the electronic device 200, the electronic device 200 may output prompt information to remind the user to wear the electronic device 200 in time. Further, the electronic device 200 may periodically identify, after step S101, whether the user wears the electronic device 200, and periodically output prompt information when the user does not wear the electronic device 200, to remind the user to wear the electronic device 200 in time.

In some implementations, when performing step S102, if the electronic device 200 identifies that the user wears the electronic device 200, the electronic device 200 may stop recording the duration recorded from the activation, to obtain the recorded duration c. The duration c is used to record the duration elapsed from the activation of the electronic device 200 to wearing of the electronic device 200. If the duration c is greater than the threshold, for example, 2 hours, the electronic device 200 is at a high risk of being infected, and is not suitable for implantation under the skin. The electronic device 200 or the electronic device 100 may output prompt information, to give the user a prompt that it is not recommended to wear the electronic device 200.

It may be understood that determining whether the duration c is greater than the threshold may be performed by the electronic device 200 or the electronic device 100. If the electronic device 200 performs determining, the electronic device 200 may perform determining in any step after step S102 and before step S107. If the electronic device 200 performs determining, the electronic device 200 may send the duration c to the electronic device 100 after step S102 and before step S107, and the electronic device 100 may determine, before step S107, whether the duration c is greater than the threshold. If the duration c is greater than the threshold, the electronic device 100 may output prompt information to remind the user that wearing the electronic device 200 is not recommended. Otherwise, step S107 may be performed.

**S108:** The electronic device 100 starts the countdown, where a countdown duration b=T-a.

Because the user has worn the electronic device 200 for a period of time before the electronic device 100 starts the countdown, the electronic device 100 does not need to wait for a complete initialization duration before outputting the body indicator data collected by the electronic device 200 in real time. In this way, waiting time of the user can be reduced, and a speed at which the user views a physical condition of the user can be increased.

For example, after obtaining the countdown duration through calculation, the electronic device 100 may display the countdown, so that the user learns about the duration in which the user needs to wait.

**S109:** The electronic device 100 outputs the body indicator data collected by the electronic device 200 in real time.

After initialization is completed, the electronic device 200 can collect the stable body indicator data of the user, and the data can be used to reflect the actual physical condition of the user. Therefore, the electronic device 200 may send the collected body indicator data to the electronic device 100, and the electronic device 100 outputs the body indicator data, so that the user learns about the current actual physical condition of the user.

It may be understood that the electronic device 100 may send, to the electronic device 200 after determining that the duration a is greater than or equal to the initialization duration T or after the countdown ends, a message for requesting to monitor the body indicator of the user, and the electronic device 200 may send, to the electronic device 100 in response to the message, the body indicator data collected in real time.

The electronic device 100 may output the body indicator data of the user in a manner like displaying, voice broadcast, or vibration. The manner in which the electronic device 100 outputs the body indicator data is not limited in embodiments of this application.

In addition, the electronic device 100 may display a value of a body indicator that is updated in real time. In this way, the user may view a current physical condition in real time, or the electronic device 100 may draw and display a continuous body indicator curve with reference to historically collected body indicator data and the body indicator data collected in real time. In this way, the user may view a fluctuation status of a body indicator of the user in a period of time. The manner in which the electronic device 100 outputs the body indicator data is not limited in embodiments of this application.

Further, the electronic device 100 may further output prompt information at preset time before the electronic device 200 expires, to prompt the user that the electronic device 200 is about to expire, so that the user wears a new device in time to monitor the body indicator of the user.

For example, a duration between the preset time and time when the electronic device 200 expires may be greater than or equal to the initialization duration T. In this way, an initialization process in which the user wears the new device configured to monitor the body indicator may be considered, so that the user can continuously monitor the physical condition of the user.

It can be learned from step S101 to step S109 that, after the user wears the electronic device 200, the duration elapsed since the user starts to wear the electronic device 200 may be recorded, so that the electronic device 100 considers time elapsed before the electronic device 100 establishes the communication connection to the electronic device 200, to omit or shorten the countdown duration of the electronic device 100, and output the body indicator data collected by the electronic device 200 as soon as possible, thereby reducing a waiting duration of the user.

It may be understood that, another device configured to monitor the body indicator of the user, for example, the electronic device 300, may also determine, based on the foregoing step S101 to step S109, a countdown waiting duration before the electronic device 100 outputs body indicator data collected by the electronic device 300.

After the electronic device 100 is simultaneously connected to a plurality of devices configured to monitor the body indicator of the user, the electronic device 100 may further identify data accuracy based on body indicator data collected by the plurality of devices.

The following describes, by using an example in which the devices simultaneously connected to the electronic device 100 include the electronic device 200 and the electronic device 300, a detailed process in which the electronic device 100 identifies accuracy of body indicator data.

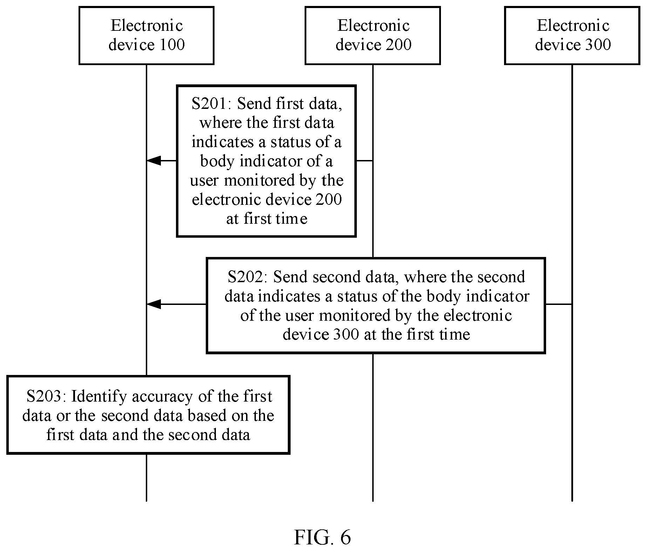

**FIG. 6** **is a schematic flowchart of a body indicator management method according to an embodiment of this application.**

**S201:** The electronic device 200 sends first data to the electronic device 100, where the first data indicates a status of a body indicator of the user monitored by the electronic device 200 at first time.

A communication connection may be established between the electronic device 100 and the electronic device 200, and the electronic device 200 may send the first data to the electronic device 100 through the communication connection.

The body indicator may be blood glucose, blood oxygen, blood ketone, lactate, blood lipid, creatinine, uric acid, blood pressure, PH, or the like.

For example, the first time may be a time period or a time point. This is not limited in embodiments of this application.

**S202:** The electronic device 300 sends second data to the electronic device 100, where the second data indicates a status of the body indicator of the user monitored by the electronic device 300 at the first time.

A communication connection may be established between the electronic device 100 and the electronic device 300, and the electronic device 300 may send the second data to the electronic device 100 through the communication connection.

The body indicator may be blood glucose, blood oxygen, blood ketone, lactate, blood lipid, creatinine, uric acid, blood pressure, PH, or the like.

For example, the electronic device 200 and the electronic device 300 each may be a CGM device, and the body indicator may be blood glucose. Alternatively, the electronic device 200 and the electronic device 300 each may be a CKM device, and the body indicator may be blood ketone. Alternatively, the electronic device 200 and the electronic device 300 each may be a CLM device, and the body indicator may be lactate.

It may be understood that step S201 may be performed first, and step S202 may be performed later; or step S202 may be performed first, and step S201 may be performed later; or step S201 and step S202 may be performed simultaneously. An execution sequence of step S201 and step S202 is not limited in this embodiment of this application.

**S203:** The electronic device 100 identifies accuracy of the first data or the second data based on the first data and the second data.

For example, time at which the user wears the electronic device 200 may be earlier than time at which the user wears the electronic device 300.

In a scenario, at an early stage of wearing the body indicator monitoring device by the user, the device is prone to early sensitivity attenuation, resulting in inaccuracy of body indicator data collected by the device. Therefore, the electronic device 100 may identify, based on the first data collected by the electronic device 200, accuracy of the second data collected by the electronic device 300.

The electronic device 100 may identify, based on a change trend of the first data, a change trend of the second data, and wearing time of the electronic device 300, whether early sensitivity attenuation occurs on the electronic device 300.

In an implementation, the electronic device 100 may determine whether a probability that early sensitivity attenuation occurs on the electronic device 200 when the electronic device 200 collects the first data is less than a threshold, whether a probability that the early sensitivity attenuation occurs on the electronic device 300 when the electronic device 300 collects the second data is greater than a threshold, and whether the first time is within a preset time period after the electronic device 300 starts to be worn, to identify whether the early sensitivity attenuation occurs on the electronic device 300.

If the probability that the early sensitivity attenuation occurs on the electronic device 200 when the electronic device 200 collects the first data is less than a first threshold, the probability that the early sensitivity attenuation occurs on the electronic device 300 when the electronic device 300 collects the second data is greater than a second threshold, and the first time is within the first preset time period after the electronic device 300 starts to be worn, it indicates that the early sensitivity attenuation occurs on the electronic device 300, and the second data is inaccurate.

For example, a probability that early sensitivity attenuation occurs on a device when the device collects data may be determined based on an attenuation amplitude of the data.

This is because the early sensitivity attenuation occurs in an early device wearing stage, and when the early sensitivity attenuation occurs on the device, the collected body indicator data shows a sharp decreasing change trend. Therefore, whether the early sensitivity attenuation occurs on the electronic device 300 may be identified by determining whether the body indicator data collected by the body indicator monitoring device shows a sharp decreasing change trend and whether wearing time of the device is in an early stage after wearing, to further reflect whether the second data is accurate.

The first threshold and the second threshold each may be a preset parameter, or one of the first threshold and the second threshold is a preset parameter, and the other parameter is determined based on the preset parameter. For example, the second threshold may be equal to the first threshold, or the second threshold may be determined based on a difference between an attenuation amplitude of the first data and the first threshold. A larger difference between the attenuation amplitude of the first data and the first threshold indicates a smaller second threshold, and a smaller difference between the attenuation amplitude of the first data and the first threshold indicates a larger second threshold. This is because if the difference between the attenuation amplitude of the first data and the first threshold is large, it indicates that there is a small possibility that an actual status of the body indicator of the user decreases sharply. Therefore, a threshold for determining that the early sensitivity attenuation occurs on the electronic device 300 may be reduced, to avoid missing identification of a possibility that the electronic device 300 collects inaccurate data. Correspondingly, if the difference between the attenuation amplitude of the first data and the first threshold is small, it indicates that there is a large possibility that the actual status of the body indicator of the user decreases sharply. Therefore, the threshold for determining that the early sensitivity attenuation occurs on the electronic device 300 may be increased, to avoid incorrectly identifying accurate data as inaccurate data.

Further, the device recovers after the early sensitivity attenuation occurs, the body indicator data collected by the device further shows a sharp increasing change trend after a sharp decrease. Therefore, whether the early sensitivity attenuation occurs on the device may be further identified based on whether an increase amplitude of the data after the decrease is greater than a threshold, to improve precision of identifying data accuracy.

It may be understood that, to further improve precision of identifying data accuracy, the electronic device 100 may extract data features of the first data and the second data. The data feature indicates a data change trend. In addition to the attenuation amplitude and the decrease amplitude mentioned above, the data feature may further include but is not limited to an average value, a maximum value, a minimum value, a quantile difference, and the like. The electronic device 100 may determine, by determining whether a data feature of the first data meets a change trend of data collected by a device on which early sensitivity attenuation occurs, the probability that the early sensitivity attenuation occurs on the electronic device 200 when the electronic device 200 collects the first data, and determine, by determining whether a data feature of the second data meets the change trend of the data collected by the device on which the early sensitivity attenuation occurs, the probability that the early sensitivity attenuation occurs on the electronic device 300 when the electronic device 300 collects the second data.

The data feature is not limited in embodiments of this application.

In another implementation, the electronic device 100 may apply, to the probability that the early sensitivity attenuation occurs on the electronic device 300 when the electronic device 300 collects the second data, the probability that the early sensitivity attenuation occurs on the electronic device 200 when the electronic device 200 collects the first data.

If it is determined that the probability that the early sensitivity attenuation occurs on the electronic device 300 when the electronic device 300 collects the second data is greater than a third threshold, and the first time is within the first preset time period after the electronic device 300 starts to be worn, it indicates that the early sensitivity attenuation occurs on the electronic device 300, and the second data is inaccurate.

The probability that the early sensitivity attenuation occurs on the electronic device 300 when the electronic device 300 collects the second data is determined based on the probability that the early sensitivity attenuation occurs on the electronic device 200 when the electronic device 200 collects the first data.

This is because the electronic device 200 and the electronic device 300 are worn at different time, and early sensitivity attenuation usually occurs in a fixed time period in which a device is worn. Therefore, early sensitivity attenuation usually does not occur on the electronic device 200 and the electronic device 300 at the same time. Further, the electronic device 200 is a device that is worn earlier than the electronic device 300, and is not prone to the early sensitivity attenuation. In comparison with the data collected by the electronic device 300, the data collected by the electronic device 200 can better reflect the actual body indicator of the user. Therefore, the probability that the early sensitivity attenuation occurs on the electronic device 300 when the electronic device 300 collects the second data may be determined based on the probability that the early sensitivity attenuation occurs on the electronic device 200 when the electronic device 200 collects the first data.

For example, if a difference between the first data and a data fluctuation trend of the early sensitivity attenuation, for example, a fluctuation trend of first sharply decreasing and then sharply increasing, is large, that is, the probability that the early sensitivity attenuation occurs on the electronic device 200 when the electronic device 200 collects the first data is small, it indicates that a possibility that an actual body indicator fluctuation of the user is similar to the data fluctuation trend of the early sensitivity attenuation is small. Therefore, when the probability that the early sensitivity attenuation occurs on the electronic device 300 when the electronic device 300 collects the second data is determined, criteria for the early sensitivity attenuation occurring on the device may be decreased. For example, even if a difference between the second data and the data fluctuation trend of the early sensitivity attenuation is large, it may be identified that the early sensitivity attenuation occurs on the electronic device 300. This can avoid missing identification of a possibility that the electronic device 300 collects inaccurate data. If the difference between the first data and the data fluctuation trend of the early sensitivity attenuation is small, that is, the probability that the early sensitivity attenuation occurs on the electronic device 200 when the electronic device 200 collects the first data is large, it indicates that the possibility that the actual body indicator fluctuation of the user is similar to the data fluctuation trend of the early sensitivity attenuation is large. Therefore, when the probability that the early sensitivity attenuation occurs on the electronic device 300 when the electronic device 300 collects the second data is determined, the criteria for the early sensitivity attenuation occurring on the device may be increased. For example, even if the difference between the second data and the data fluctuation trend of the early sensitivity attenuation is small, it may not be identified that the early sensitivity attenuation occurs on the electronic device 300. This can avoid incorrectly identifying accurate data collected by the electronic device 300 as inaccurate data.

In a specific implementation, whether the data feature of the first data meets the change trend of the data collected by the device on which the early sensitivity attenuation occurs may be determined by extracting the data feature of the first data, to determine the probability that the early sensitivity attenuation occurs on the electronic device 200 when the electronic device 200 collects the first data. In addition, whether the data feature of the second data meets the change trend of the data collected by the device on which the early sensitivity attenuation occurs may be determined by extracting the data feature of the second data and changing a weight of the data feature of the second data based on the probability that the early sensitivity attenuation occurs on the electronic device 200 when the electronic device 200 collects the first data, to determine the probability that the early sensitivity attenuation occurs on the electronic device 300 when the electronic device 300 collects the second data.

In addition, when it is determined whether the first time is within the preset time period after the electronic device 300 starts to be worn, the preset time period may have the following two cases:
(1) The preset time period may be a specified time period determined by a developer.
   For example, the preset time period is the first 12 to 24 hours when the device is worn. This is because the early sensitivity attenuation usually occurs in the first 12 to 24 hours when the device is worn. Therefore, whether the early sensitivity attenuation occurs on the electronic device 300 may be identified with reference to determining whether a wearing duration of the electronic device 300 is within a time period in which the early sensitivity attenuation generally occurs, resulting in inaccuracy of data collected by the electronic device 300.
   It may be understood that time periods in which early sensitivity attenuation occurs on different devices may be different. The developer may test in advance time periods in which early sensitivity attenuation occurs on devices produced by different manufacturers, to determine the preset time period. The preset time period is not limited in embodiments of this application.
(2) The preset time period is a time period determined based on historical body indicator data.

This is because early sensitivity attenuation is related to a physique of the user. After the user wears a device, a time period in which early sensitivity attenuation occurs is usually fixed. Therefore, the electronic device 100 may determine, based on the historical body indicator data, time periods in which early sensitivity attenuation occurs on body indicator monitoring devices that are historically worn, and determine the preset time period based on these time periods. For example, a time period with the highest occurrence frequency in these historical time periods is selected as the preset time period.

It may be understood that the preset time period may be alternatively determined in another manner. This is not limited in embodiments of this application.

In another scenario, at a late stage of wearing the body indicator monitoring device by the user, the device is prone to late sensor attenuation, resulting in inaccuracy of body indicator data collected by the device. Therefore, the electronic device 100 may identify, based on the second data collected by the electronic device 300, accuracy of the first data collected by the electronic device 200.

The electronic device 100 may identify, based on a change trend of the first data, a change trend of the second data, and wearing time of the electronic device 200, whether late sensor attenuation occurs on the electronic device 200.

In an implementation, the electronic device 100 may determine whether a probability that the late sensor attenuation occurs on the electronic device 200 when the electronic device 200 collects the first data is greater than a threshold, whether a probability that late sensor attenuation occurs on the electronic device 300 when the electronic device 300 collects the second data is less than a threshold, and whether the first time is within a preset time period before the electronic device 200 expires, to identify whether the late sensor attenuation occurs on the electronic device 200.

If the probability that the late sensor attenuation occurs on the electronic device 200 when the electronic device 200 collects the first data is greater than a fourth threshold, the probability that the late sensor attenuation occurs on the electronic device 300 when the electronic device 300 collects the second data is less than a fifth threshold, and the first time is within a second preset time period before the electronic device 200 expires, it indicates that the late sensor attenuation occurs on the electronic device 200, and the first data is inaccurate.

For example, a probability that late sensor attenuation occurs on a device when the device collects data may be determined based on an attenuation amplitude of the data.

This is because the late sensor attenuation occurs in a late stage of wearing the device, and when the late sensor attenuation occurs on the device, the collected body indicator data shows a sharp decreasing change trend. Therefore, whether the late sensor attenuation occurs on the electronic device 200 may be identified by determining whether the body indicator data collected by the body indicator monitoring device shows a sharp decreasing change trend and whether wearing time of the device is in a late stage of wearing, to further reflect whether the first data is accurate.

The fourth threshold and the fifth threshold each may be a preset parameter, or one of the fourth threshold and the fifth threshold is a preset parameter, and the other parameter is determined based on the preset parameter. For example, the fourth threshold may be equal to the fifth threshold, or the fourth threshold may be determined based on a difference between an attenuation amplitude of the second data and the fifth threshold. A larger difference between the attenuation amplitude of the second data and the fourth threshold indicates a smaller fifth threshold, and a smaller difference between the attenuation amplitude of the second data and the fifth threshold indicates a larger fourth threshold. This is because if the difference between the attenuation amplitude of the second data and the fifth threshold is large, it indicates that there is a small possibility that the actual status of the body indicator of the user decreases sharply. Therefore, a threshold for determining that the late sensor attenuation occurs on the electronic device 200 may be reduced, to avoid missing identification of a possibility that the electronic device 200 collects inaccurate data. Correspondingly, if the difference between the attenuation amplitude of the second data and the fifth threshold is small, it indicates that there is a large possibility that the actual status of the body indicator of the user decreases sharply. Therefore, the threshold for determining that the late sensor attenuation occurs on the electronic device 200 may be increased, to avoid incorrectly identifying accurate data as inaccurate data.

Further, because the device does not recover after the late sensor attenuation occurs, the electronic device 100 may identify accuracy of the data based on the data collected by the electronic device 200 and the data collected by the electronic device 300 at night. This is because the user has few exercise at night, and a possibility of a fluctuation of the body indicator of the user is small. Because the data collected by the device during the late sensor attenuation continuously decreases, it is easier to identify, based on the data collected at night, whether the late sensor attenuation occurs on the device.

It may be understood that the electronic device 100 may further determine, with reference to another factor, whether the late sensor attenuation occurs on the device. This is not limited in embodiments of this application.

In another implementation, the electronic device 100 may apply, to the probability that the late sensor attenuation occurs on the electronic device 200 when the electronic device 200 collects the first data, the probability that the late sensor attenuation occurs on the electronic device 300 when the electronic device 300 collects the second data.

If it is determined that the probability that the late sensor attenuation occurs on the electronic device 200 when the electronic device 200 collects the first data is greater than a sixth threshold, and the first time is within a second preset time period before the electronic device 200 expires, it indicates that the late sensor attenuation occurs on the electronic device 200, and the first data is inaccurate.

The probability that the late sensor attenuation occurs on the electronic device 200 when the electronic device 200 collects the first data is determined based on the probability that the late sensor attenuation occurs on the electronic device 300 when the electronic device 300 collects the second data.

This is because the electronic device 200 and the electronic device 300 are worn at different time, and late sensor attenuation usually occurs in a fixed time period in which a device is worn. Therefore, late sensor attenuation usually does not occur on the electronic device 200 and the electronic device 300 at the same time. Further, the electronic device 300 is a device that is worn later than the electronic device 200, and is not prone to the late sensor attenuation. In comparison with the data collected by the electronic device 200, the data collected by the electronic device 300 can better reflect the actual body indicator of the user. Therefore, the probability that the late sensor attenuation occurs on the electronic device 200 when the electronic device 200 collects the first data may be determined based on the probability that the late sensor attenuation occurs on the electronic device 300 when the electronic device 300 collects the second data.

For example, if a difference between the second data and a data fluctuation trend of the late sensor attenuation, for example, a fluctuation trend of sharply decreasing, is large, that is, the probability that the late sensor attenuation occurs on the electronic device 300 when the electronic device 300 collects the second data is small, it indicates that a possibility that an actual body indicator fluctuation of the user is similar to the data fluctuation trend of the late sensor attenuation is small. Therefore, when the probability that the late sensor attenuation occurs on the electronic device 200 when the electronic device 200 collects the first data is determined, criteria for the late sensor attenuation occurring on the device may be decreased. For example, even if a difference between the first data and the data fluctuation trend of the late sensor attenuation is large, it may be identified that the late sensor attenuation occurs on the electronic device 200. This can avoid missing identification of a possibility that the electronic device 200 collects inaccurate data. If the difference between the second data and the data fluctuation trend of the late sensor attenuation is small, that is, the probability that the late sensor attenuation occurs on the electronic device 300 when the electronic device 300 collects the second data is large, it indicates that the possibility that the actual body indicator fluctuation of the user is similar to the data fluctuation trend of the late sensor attenuation is large. Therefore, when the probability that the late sensor attenuation occurs on the electronic device 200 when the electronic device 200 collects the first data is determined, the criteria for the late sensor attenuation occurring on the device may be increased. For example, even if the difference between the first data and the data fluctuation trend of the late sensor attenuation is small, it may not be identified that the late sensor attenuation occurs on the electronic device 200. This can avoid incorrectly identifying accurate data collected by the electronic device 200 as inaccurate data.

In a specific implementation, whether the data feature of the second data meets the change trend of the data collected by the device on which the late sensor attenuation occurs may be determined by extracting the data feature of the second data, to determine the probability that the late sensor attenuation occurs on the electronic device 300 when the electronic device 300 collects the second data. In addition, whether the data feature of the second data meets the change trend of the data collected by the device on which the late sensor attenuation occurs may be determined by extracting the data feature of the first data and changing a weight of the data feature of the second data based on the probability that the late sensor attenuation occurs on the electronic device 300 when the electronic device 300 collects the second data, to determine the probability that the late sensor attenuation occurs on the electronic device 200 when the electronic device 200 collects the first data.

In addition, when it is determined whether the first time is within the preset time period before the electronic device 200 expires, the preset time period may have the following two cases:
(1) The preset time period may be a specified time period determined by the developer.
   For example, the preset time period is the last 12 to 24 hours when the device is worn. This is because the late sensor attenuation usually occurs in the last 12 to 24 hours when the device is worn. Therefore, whether the late sensor attenuation occurs on the electronic device 200 may be identified with reference to determining whether a wearing duration of the electronic device 200 is within a time period in which the late sensor attenuation generally occurs, resulting in inaccuracy of data collected by the electronic device 200.
   It may be understood that time periods in which late sensor attenuation occurs on different devices may be different. The developer may test in advance time periods in which late sensor attenuation occurs on devices produced by different manufacturers, to determine the preset time period. The preset time period is not limited in embodiments of this application.
(2) The preset time period is a time period determined based on historical body indicator data.

This is because late sensor attenuation is related to a physique of the user. After the user wears a device, a time period in which late sensor attenuation occurs is usually fixed. Therefore, the electronic device 100 may determine, based on the historical body indicator data, time periods in which late sensor attenuation occurs on body indicator monitoring devices that are historically worn, and determine the preset time period based on these time periods. For example, a time period with the highest occurrence frequency in these historical time periods is selected as the preset time period.

It may be understood that the preset time period may be alternatively determined in another manner. This is not limited in embodiments of this application.

In addition, it should be noted that, in addition to inaccuracy of body indicator data collected by a device due to early sensitivity attenuation or late sensor attenuation occurring on the device, if the device is collided by an external object, is not worn in a standard manner, or the like, body indicator data collected by the device may be inaccurate.

In a scenario, if a body indicator monitoring device is squeezed when the user wears the body indicator monitoring device, body indicator data collected by the device may be inaccurate. Therefore, the electronic device 100 may identify, based on the first data collected by the electronic device 200, accuracy of the second data collected by the electronic device 300, or identify, based on the second data collected by the electronic device 300, accuracy of the first data collected by the electronic device 200.

If the electronic device 100 identifies, based on the first data and the second data, that the electronic device 200 is squeezed at the first time, the electronic device 100 determines that the first data is inaccurate. If the electronic device 100 identifies, based on the first data and the second data, that the electronic device 300 is squeezed at the first time, the electronic device 100 determines that the second data is inaccurate.

The electronic device 100 may identify, based on the change trend of the first data and the change trend of the second data, whether the electronic device 200 or the electronic device 300 is squeezed.

A CGM device is used as an example. If the CGM device is squeezed, a blood glucose value collected by the CGM device shows a change trend of first decreasing and then increasing. Therefore, whether the electronic device 200 or the electronic device 300 is squeezed may be identified based on whether the first data and the second data show a change trend of first decreasing and then increasing.

Further, the electronic device 100 may further identify, with reference to a change trend of other data, whether the electronic device 200 or the electronic device 300 is squeezed.

A CGM device is used as an example. If the CGM device is squeezed, a temperature value collected by the CGM device shows a change trend of first increasing and then decreasing. Therefore, whether the electronic device 200 or the electronic device 300 is squeezed may be identified with reference to data reflecting a body indicator, for example, the first data or the second data, and the change trend of the temperature value.

It can be learned that the electronic device 100 may further identify the accuracy of the first data or the accuracy of the second data with reference to other physiological data related to the body indicator of the user. The physiological data may include but is not limited to one or more of the following: blood pressure, a heart rate, a pulse, a skin temperature, and the like. For example, the electronic device 100 may identify the accuracy of the second data with reference to whether a change amplitude of the other physiological data with time is greater than a threshold. For example, the body indicator monitored by the electronic device 200 and the electronic device 300 is blood glucose. The other physiological data may be the heart rate. Generally, when the blood glucose of the user is abnormal, the heart rate is also abnormal. Therefore, if a change amplitude of the blood glucose monitored by the electronic device 200 is greater than a threshold, and a change amplitude of the blood glucose monitored by the electronic device 300 is less than the threshold, it indicates that the electronic device 200 or the electronic device 300 has a problem of inaccurate monitoring. If a change amplitude of the heart rate of the user is greater than a threshold, it indicates that the blood glucose of the user currently fluctuates greatly, and there is indeed a phenomenon of abnormal blood glucose. Therefore, it indicates that the blood glucose value collected by the electronic device 300 is inaccurate. If the change amplitude of the heart rate of the user is less than the threshold, it indicates that the heart rate of the user currently fluctuates slightly, and there is no phenomenon of abnormal blood glucose. Therefore, it indicates that the blood glucose value collected by the electronic device 200 is inaccurate.

It may be understood that the electronic device 100 may further identify the accuracy of the first data or the accuracy of the second data with reference to another factor. The factor may include exercise of the user, a food intake amount, a sleep status, a body movement of the user, and the like.

For example, when the accuracy of the first data or the accuracy of the second data is identified with reference to the exercise of the user, whether the user has a phenomenon that a change amplitude of the body indicator of the user is large because the user performs an excessively large amount of exercise may be determined based on the exercise of the user. If the phenomenon does not exist, it indicates that data with a large change amplitude in the first data and the second data is inaccurate data. If the phenomenon exists, it indicates that data with a small change amplitude in the first data and the second data is inaccurate data.

For another example, when the accuracy of the first data or the accuracy of the second data is identified with reference to the food intake amount of the user, whether the user has a phenomenon that a change amplitude of the body indicator of the user is large due to an irregular diet of the user may be determined based on the food intake amount of the user. If the phenomenon does not exist, it indicates that data with a large change amplitude in the first data or the second data is inaccurate data. If the phenomenon exists, it indicates that data with a small change amplitude in the first data or the second data is inaccurate data.

In some implementations, after the electronic device 100 identifies the accuracy of the first data and the accuracy of the second data, the electronic device 100 may output first prompt information, where the first prompt information is used to give a prompt that the first data or the second data is inaccurate. This is to remind the user to distinguish between accurate and inaccurate body indicator data monitored by the devices.

In some implementations, after the electronic device 100 obtains the first data and the second data, the electronic device 100 may further display the first data and the second data, so that the user learns about a monitoring status of the body indicator monitoring device for the body indicator of the user.

For example, the electronic device 100 may display, in real time, a body indicator value collected by the electronic device 200 or the electronic device 300, or may display a curve drawn based on body indicator data collected by the electronic device 200 and/or the electronic device 300. Display forms of the first data and the second data are not limited in embodiments of this application.

A manner in which the electronic device 100 displays the first data and the second data may include any one or more of the following:
(1) The electronic device 100 displays the first data or the second data.

In other words, the electronic device 100 may display body indicator data collected by one body indicator monitoring device, so that the user focuses on the body indicator data monitored by the device.

For example, the electronic device 100 may display a curve drawn based on the first data or a curve drawn based on the second data.

In addition, when the electronic device 100 displays the curve drawn based on the first data or the curve drawn based on the second data, the electronic device 100 may distinguish data with different accuracy in the curve. For example, if the first time includes a first time period, and data in the second data in the first time period is inaccurate, when the electronic device 100 displays the curve drawn based on the second data, a curve segment corresponding to the first time period in the curve may be highlighted, so that the user distinguishes body indicator data with different accuracy.

Alternatively, when the electronic device 100 displays the curve drawn based on the first data or the curve drawn based on the second data, the electronic device 100 may display a curve drawn based on accurate data, and does not display a curve drawn based on inaccurate data, or the electronic device 100 may display a curve drawn based on inaccurate data, and does not display a curve drawn based on accurate data.

Further, the electronic device 100 may further change, based on a user operation, the curve displayed by the electronic device 100. For example, if the electronic device 100 displays the curve drawn based on the second data, the electronic device 100 may detect a user operation, for example, a first operation, to update the curve, where the second data includes the inaccurate data in the first time period and accurate data in a second time period, the displayed curve may include a curve segment corresponding to the second time period, and does not include the curve segment corresponding to the first time period, and the updated curve may include both the curve segment corresponding to the first time period and the curve segment corresponding to the second time period.

In addition, the electronic device 100 may further display exception information, to prompt a user with a reason why the data is inaccurate. The reason may be early sensitivity attenuation, late sensor attenuation, device squeezing, user exercise impact, user mood impact, or the like. For example, if the data in the second data in the first time period is inaccurate, the electronic device 100 may display first exception information, where the first exception information may indicate a reason why the data in the second data in the first time period is inaccurate.

The electronic device 100 may select, in any one of the following manners, a device configured to display the body indicator data:
(a) After a body indicator monitoring device (for example, the electronic device 200) expires, the electronic device 100 may switch to display body indicator data collected by another body indicator monitoring device (for example, the electronic device 300).
(b) The electronic device 100 may switch to display, after establishing a communication connection to another body indicator monitoring device (for example, the electronic device 300), body indicator data collected by the body indicator monitoring device.
(c) The electronic device 100 may switch to display, when currently displayed body indicator data is inaccurate, body indicator data collected by another body indicator monitoring device.
(d) The electronic device 100 selects, based on a user operation, a device whose body indicator data is to be displayed.

For example, FIG. 2D shows that the electronic device 100 displays, under the selection of the user, the body indicator data collected by the electronic device 200.

(2) The electronic device 100 simultaneously displays the first data and the second data.

For example, the electronic device 100 may display both a curve drawn based on the first data and a curve drawn based on the second data.

In other words, the electronic device 100 may simultaneously display body indicator data collected by a plurality of body indicator monitoring devices, so that the user can simultaneously view monitoring statuses of different devices for the body indicator of the user.

For example, FIG. 2E(a) shows that the electronic device 100 displays, in a same coordinate system, curves that are drawn based on body indicator data collected by the electronic device 200 and the electronic device 300, and FIG. 2E(b) shows that the electronic device 100 displays, respectively in different coordinate systems, curves that are drawn based on the body indicator data collected by the electronic device 200 and the electronic device 300.

(3) The electronic device 100 displays third data obtained based on the first data and the second data.

For example, the electronic device 100 may display a curve drawn based on the first data and the second data.

The third data may be accurate data in the first data or the second data, or the third data is determined based on accurate data in the first data and the second data at the same time.

For example, if the first time includes a first time period and a second time period, the electronic device 100 identifies that data in the first data in the first time period and data in the first data in the second time period are accurate, data in the second data in the first time period is inaccurate, and data in the second data in the second time period is inaccurate, the curve drawn by the electronic device 100 based on the first data and the second data may include: a curve segment drawn based on the data in the first data in the first time period, and a curve segment drawn based on the third data, where the third data is the data in the first data or the second data in the second time period, or the third data is determined based on the data in the first data and the data in the second data in the second time period.

For example, FIG. 2E(c) shows a curve that is displayed by the electronic device 100 and that is drawn based on data obtained from the body indicator data collected by the electronic device 100 and the electronic device 200.

In some implementations, after the electronic device 100 identifies the accuracy of the first data and the accuracy of the second data, the electronic device 100 may further evaluate the body indicator of the user based on fourth data. The fourth data may include accurate data in the first data, and does not include inaccurate data in the first data.

Further, optionally, the fourth data may further include the accurate data in the second data, and does not include the inaccurate data in the second data.

In this way, the electronic device 100 can be prevented from using inaccurate data to evaluate the body indicator of the user, thereby improving accuracy of evaluating the body condition of the user.

In some implementations, the electronic device 100 may further display data in the first data and data in the second data according to different abnormality classifications. The abnormality may include early sensitivity attenuation, late sensor attenuation, sleep abnormality, excessive exercise, irregular diet, bathing, sitting for a long time, partial device falling off, and the like. These abnormalities may be identified based on the first data and the second data, and in combination with other factors, for example, device wearing time, a user sleep status, exercise, a diet condition, and body movement.

In addition, the electronic device 100 may further display, based on a user operation, body indicator data corresponding to an abnormality selected by the user, so that the user can learn about, in a targeted manner, statuses of the body indicator monitored by the device in different abnormality cases.

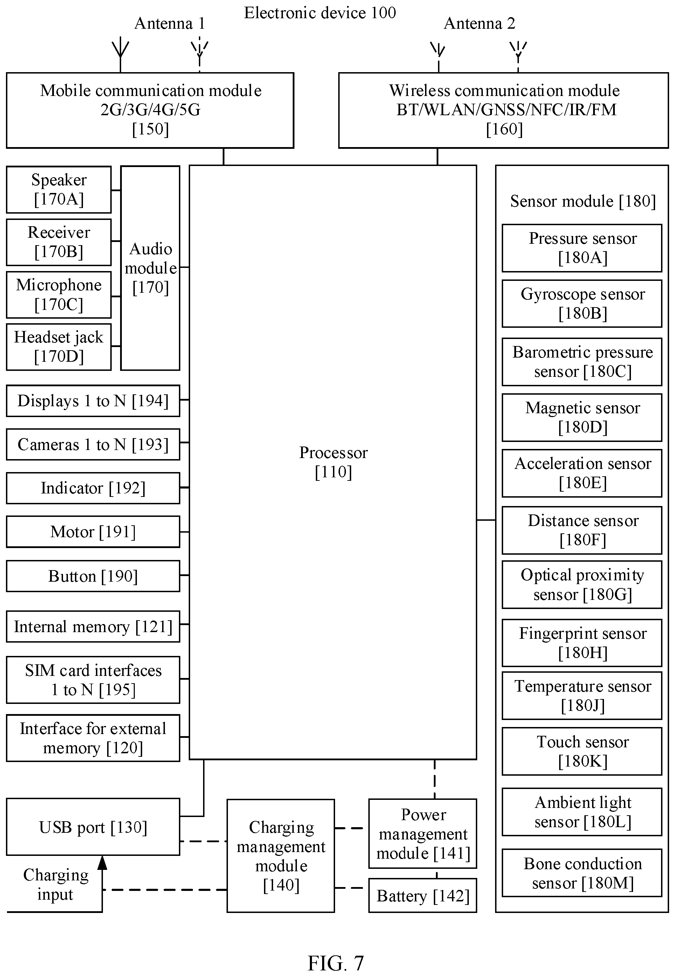

**FIG. 7** **is a diagram of a hardware structure of the electronic device 100.**

The electronic device 100 may be a mobile phone, a tablet computer, a desktop computer, a laptop computer, a handheld computer, a notebook computer, an ultra-mobile personal computer (ultra-mobile personal computer, UMPC), a netbook, a cellular phone, a personal digital assistant (personal digital assistant, PDA), an augmented reality (augmented reality, AR) device, a virtual reality (virtual reality, VR) device, an artificial intelligence (artificial intelligence, AI) device, a wearable device, a vehicle-mounted device, a smart home device, and/or a smart city device. A specific type of the electronic device is not limited in embodiments of this application.

The electronic device 100 may include a processor 110, an interface 120 for external memory, an internal memory 121, a universal serial bus (universal serial bus, USB) port 130, a charging management module 140, a power management module 141, a battery 142, an antenna 1, an antenna 2, a mobile communication module 150, a wireless communication module 160, an audio module 170, a speaker 170A, a receiver 170B, a microphone 170C, a headset jack 170D, a sensor module 180, a button 190, a motor 191, an indicator 192, a camera 193, a display 194, a subscriber identification module (subscriber identification module, SIM) card interface 195, and the like. The sensor module 180 may include a pressure sensor 180A, a gyroscope sensor 180B, a barometric pressure sensor 180C, a magnetic sensor 180D, an acceleration sensor 180E, a distance sensor 180F, an optical proximity sensor 180G, a fingerprint sensor 180H, a temperature sensor 180J, a touch sensor 180K, an ambient light sensor 180L, a bone conduction sensor 180M, and the like.

It may be understood that the structure shown in embodiments of the present invention does not constitute a specific limitation on the electronic device 100. In some other embodiments of this application, the electronic device 100 may include more or fewer components than those shown in the figure, or some components may be combined, or some components may be split, or different component arrangements may be used. The components shown in the figure may be implemented by hardware, software, or a combination of software and hardware.

The processor 110 may include one or more processing units. For example, the processor 110 may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, a neural-network processing unit (neural-network processing unit, NPU), and/or the like. Different processing units may be independent devices, or may be integrated into one or more processors.

**In some implementations, the processor 110 may be configured to identify, based on body indicator data collected by the electronic device 200 and body indicator data collected by the electronic device 300, accuracy of a body indicator collected by one of the devices.**

The controller may generate an operation control signal based on an instruction operation code and a time sequence signal, to complete control of instruction reading and instruction execution.

A memory may be further disposed in the processor 110, and is configured to store instructions and data. In some embodiments, the memory in the processor 110 is a cache memory. The memory may store instructions or data just used or cyclically used by the processor 110. If the processor 110 needs to use the instructions or the data again, the processor may directly invoke the instructions or the data from the memory. This avoids repeated access, reduces waiting time of the processor 110, and improves system efficiency.

A wireless communication function of the electronic device 100 may be implemented through the antenna 1, the antenna 2, the mobile communication module 150, the wireless communication module 160, the modem processor, the baseband processor, and the like.

The antenna 1 and the antenna 2 are configured to transmit and receive an electromagnetic wave signal. Each antenna in the electronic device 100 may be configured to cover one or more communication frequency bands. Different antennas may be further reused, to improve antenna utilization. For example, the antenna 1 may be reused as a diversity antenna of a wireless local area network. In some other embodiments, the antenna may be used in combination with a tuning switch.

The mobile communication module 150 may provide a wireless communication solution that includes 2G/3G/4G/5G or the like and that is applied to the electronic device 100. The mobile communication module 150 may include at least one filter, a switch, a power amplifier, a low noise amplifier (low noise amplifier, LNA), and the like. The mobile communication module 150 may receive an electromagnetic wave through the antenna 1, perform processing such as filtering or amplification on the received electromagnetic wave, and transmit the electromagnetic wave to the modem processor for demodulation. The mobile communication module 150 may further amplify a signal modulated by the modem processor, and convert the signal into an electromagnetic wave for radiation through the antenna 1. In some embodiments, at least some functional modules in the mobile communication module 150 may be disposed in the processor 110. In some embodiments, at least some functional modules of the mobile communication module 150 may be disposed in a same device as at least some modules of the processor 110.

The modem processor may include a modulator and a demodulator. The modulator is configured to modulate a to-be-sent low-frequency baseband signal into a medium-high frequency signal. The demodulator is configured to demodulate a received electromagnetic wave signal into a low-frequency baseband signal. Then, the demodulator transmits the low-frequency baseband signal obtained through demodulation to the baseband processor for processing. The low-frequency baseband signal is processed by the baseband processor and then transmitted to the application processor. The application processor outputs a sound signal through an audio device (which is not limited to the speaker 170A, the receiver 170B, or the like), or displays an image or a video through the display 194. In some embodiments, the modem processor may be an independent device. In some other embodiments, the modem processor may be independent of the processor 110, and is disposed in a same device as the mobile communication module 150 or another functional module.

The wireless communication module 160 may provide a wireless communication solution that is applied to the electronic device 100 and that includes a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, or the like. The wireless communication module 160 may be one or more components integrating at least one communication processor module. The wireless communication module 160 receives an electromagnetic wave through the antenna 2, performs demodulation and filtering on an electromagnetic wave signal, and sends a processed signal to the processor 110. The wireless communication module 160 may further receive a to-be-sent signal from the processor 110, perform frequency modulation and amplification on the signal, and convert the signal into an electromagnetic wave for radiation through the antenna 2.

In some embodiments, the antenna 1 and the mobile communication module 150 in the electronic device 100 are coupled, and the antenna 2 and the wireless communication module 160 in the electronic device 100 are coupled, so that the electronic device 100 can communicate with a network and another device by using a wireless communication technology. The wireless communication technology may include a global system for mobile communications (global system for mobile communications, GSM), a general packet radio service (general packet radio service, GPRS), code division multiple access (code division multiple access, CDMA), wideband code division multiple access (wideband code division multiple access, WCDMA), time-division code division multiple access (time-division code division multiple access, TD-SCDMA), long term evolution (long term evolution, LTE), BT, a GNSS, a WLAN, NFC, FM, an IR technology, and/or the like. The GNSS may include a global positioning system (global positioning system, GPS), a global navigation satellite system (global navigation satellite system, GLONASS), a BeiDou navigation satellite system (BeiDou navigation satellite system, BDS), a quasi-zenith satellite system (quasi-zenith satellite system, QZSS), and/or satellite based augmentation systems (satellite based augmentation systems, SBAS).

**In some implementations, the electronic device 100 may receive, by using the mobile communication module 150 or the wireless communication module 160, the body indicator data collected by the electronic device 200 and the body indicator data collected by the electronic device 300.**

The electronic device 100 may implement a display function through the GPU, the display 194, the application processor, and the like. The GPU is a microprocessor for image processing, and is connected to the display 194 and the application processor. The GPU is configured to: perform mathematical and geometric computation, and render an image. The processor 110 may include one or more GPUs, which execute program instructions to generate or change display information.

The display 194 is configured to display an image, a video, and the like. In some embodiments, the electronic device may include one or N displays 194, where N is a positive integer greater than 1.

**In some implementations, the display 194 may be configured to display the body indicator data collected by the electronic device 200 and the body indicator data collected by the electronic device 300.**

**For details about a user interface displayed on the display 194, refer to** **FIG. 2A to FIG. 2E(c)** **and** **FIG. 3A to FIG. 3F****. Details are not described herein again.**

The electronic device 100 may implement an image shooting function through the ISP, the camera 193, the video codec, the GPU, the display 194, the application processor, and the like.

The camera 193 is configured to capture a static image or a video. In some embodiments, the electronic device 100 may include one or N cameras 193, where N is a positive integer greater than 1.

The internal memory 121 may include one or more random access memories (random access memories, RAMs) and one or more non-volatile memories (non-volatile memories, NVMs).

The random access memory may be directly read and written by using the processor 110. The random access memory may be configured to store an executable program (for example, machine instructions) in an operating system or another running program, and may be further configured to store data of a user, data of an application, and the like.

The non-volatile memory may also store an executable program, data of a user, data of an application, and the like, which may be loaded into the random access memory in advance for directly reading and writing by the processor 110.

**In some implementations, the internal memory 121 may be configured to store body indicator data obtained by the electronic device 100.**

The electronic device 100 may implement an audio function, for example, music playing and recording, through the audio module 170, the speaker 170A, the receiver 170B, the microphone 170C, the headset jack 170D, the application processor, and the like.

The gyroscope sensor 180B may be configured to determine a moving posture of the electronic device 100. In some embodiments, an angular velocity of the electronic device 100 around three axes (namely, axes x, y, and z) may be determined by using the gyroscope sensor 180B. The gyroscope sensor 180B may be configured to implement image stabilization during image shooting. For example, when a shutter is pressed, the gyroscope sensor 180B detects an angle at which the electronic device 100 jitters, calculates, based on the angle, a distance for which a lens module needs to compensate, and allows the lens to cancel the jitter of the electronic device 100 through reverse motion, to implement image stabilization. The gyroscope sensor 180B may also be used in a navigation scenario and a somatic game scenario.

The acceleration sensor 180E may detect accelerations in various directions (usually on three axes) of the electronic device 100. The acceleration sensor may detect a magnitude and a direction of gravity when the electronic device 100 is still. The acceleration sensor may be further configured to identify a posture of the electronic device, and is used in an application, for example, switching between a landscape mode and a portrait mode or a pedometer.

The touch sensor 180K is also referred to as a "touch component". The touch sensor 180K may be disposed on the display 194, and the touch sensor 180K and the display 194 constitute a touchscreen, which is also referred to as a "touch screen". The touch sensor 180K is configured to detect a touch operation performed on or near the touch sensor. The touch sensor may transfer the detected touch operation to the application processor to determine a type of the touch event. The display 194 may provide a visual output related to the touch operation. In some other embodiments, the touch sensor 180K may also be disposed on a surface of the electronic device 100 at a location different from that of the display 194.

The electronic device may be a portable terminal device carrying Harmony, iOS, Android, Microsoft, or another operating system, for example, a mobile phone, a tablet computer, or a wearable device; or may be a non-portable terminal device, for example, a laptop (Laptop) computer having a touch-sensitive surface or a touch panel, or a desktop computer having a touch-sensitive surface or a touch panel. A software system of the electronic device 100 may use a layered architecture, an event-driven architecture, a microkernel architecture, a micro service architecture, or a cloud architecture. In this embodiment of the present invention, an Android system of a layered architecture is used as an example to illustrate the software structure of the electronic device 100.

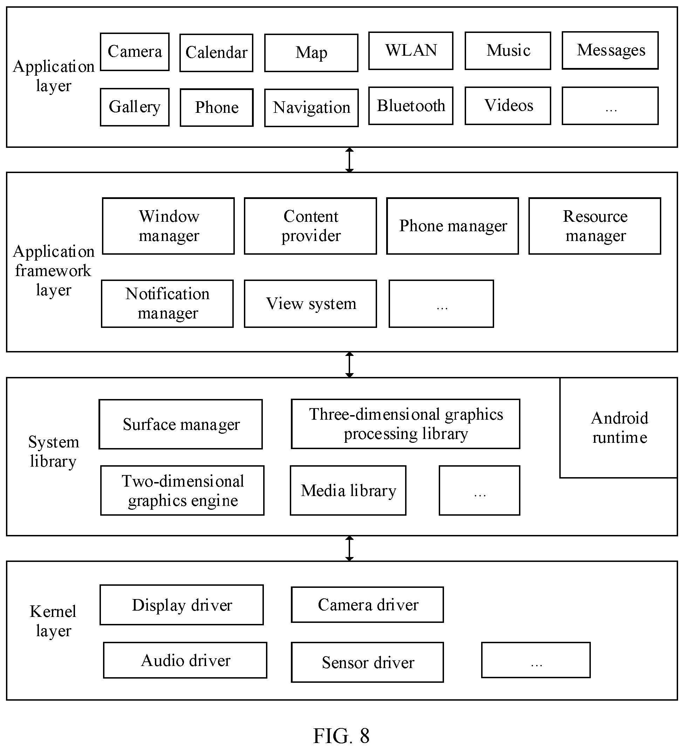

**FIG. 8** **is a block diagram of the software structure of the electronic device 100 according to an embodiment of this application.**

In a layered architecture, software is divided into several layers, and each layer has a clear role and task. The layers communicate with each other through a software interface. In some embodiments, the Android system is divided into four layers: an application layer, an application framework layer, an Android runtime (Android runtime) and system library, and a kernel layer from top to bottom.

The application layer may include a series of application packages.

As shown in FIG. 8, the application packages may include applications such as Camera, Gallery, Calendar, Phone, Map, Navigation, WLAN, Bluetooth, Music, Videos, and Messages.

The application framework layer provides an application programming interface (application programming interface, API) and a programming framework for an application at the application layer. The application framework layer includes some predefined functions.

As shown in FIG. 8, the application framework layer may include a window manager, a content provider, a view system, a phone manager, a resource manager, a notification manager, and the like.

The window manager is configured to manage a window program. The window manager may obtain a size of the display, determine whether there is a status bar, perform screen locking, take a screenshot, and the like.

The content provider is configured to: store and obtain data, and enable the data to be accessed by an application. The data may include a video, an image, an audio, calls that are made and answered, a browsing history and bookmarks, an address book, and the like.

The view system includes visual controls such as a control for displaying a text and a control for displaying a picture. The view system may be configured to construct an application. A display interface may include one or more views. For example, a display interface including a notification icon of Messages may include a text display view and an image display view.

The phone manager is configured to provide a communication function for the electronic device 100, for example, management of a call status (including answering, declining, or the like).

The resource manager provides various resources such as a localized character string, an icon, a picture, a layout file, and a video file for an application.

The notification manager enables an application to display notification information in a status bar, and may be configured to convey a notification message. The displayed notification information may automatically disappear after a short pause without requiring a user interaction. For example, the notification manager is configured to notify download completion, give a message notification, and the like. The notification manager may alternatively be a notification that appears in a top status bar of the system in a form of a graph or a scroll bar text, for example, a notification of an application that is run on a background, or may be a notification that appears on a screen in a form of a dialog window. For example, text information is prompted in the status bar, an alert tone is made, the electronic device vibrates, or an indicator blinks.

The Android runtime includes a core library and a virtual machine. The Android runtime is responsible for scheduling and management of the Android system.

The core library includes two parts: a function that needs to be invoked in Java language, and a core library of Android.

The application layer and the application framework layer run on the virtual machine. The virtual machine executes Java files of the application layer and the application framework layer as binary files. The virtual machine is configured to perform functions such as object lifecycle management, stack management, thread management, security and exception management, and garbage collection.

The system library may include a plurality of functional modules, for example, a surface manager (surface manager), a media library (Media Library), a three-dimensional graphics processing library (for example, OpenGL ES), and a 2D graphics engine (for example, SGL).

The surface manager is configured to manage a display subsystem and provide fusion of 2D and 3D layers for a plurality of applications.

The media library supports playback and recording in a plurality of commonly used audio and video formats, static image files, and the like. The media library may support a plurality of audio and video coding formats, for example, MPEG-4, H.264, MP3, AAC, AMR, JPG, and PNG.

The three-dimensional graphics processing library is configured to implement three-dimensional graphics drawing, image rendering, composition, layer processing, and the like.

The 2D graphics engine is a drawing engine for 2D drawing.

The kernel layer is a layer between hardware and software. The kernel layer includes at least a display driver, a camera driver, an audio driver, and a sensor driver.

The following describes an example of a working process of software and hardware of the electronic device 100 with reference to a photographing scenario.

When the touch sensor 180K receives a touch operation, a corresponding hardware interrupt is sent to the kernel layer. The kernel layer processes the touch operation into an original input event (including information such as touch coordinates and a time stamp of the touch operation). The original input event is stored at the kernel layer. The application framework layer obtains the original input event from the kernel layer, and identifies a control corresponding to the input event. An example in which the touch operation is a touch operation, and a control corresponding to the touch operation is a control of a camera application icon is used. A camera application invokes an interface of the application framework layer to start the camera application, so that the camera driver is started by invoking the kernel layer, and a static image or a video is captured through the camera 193.

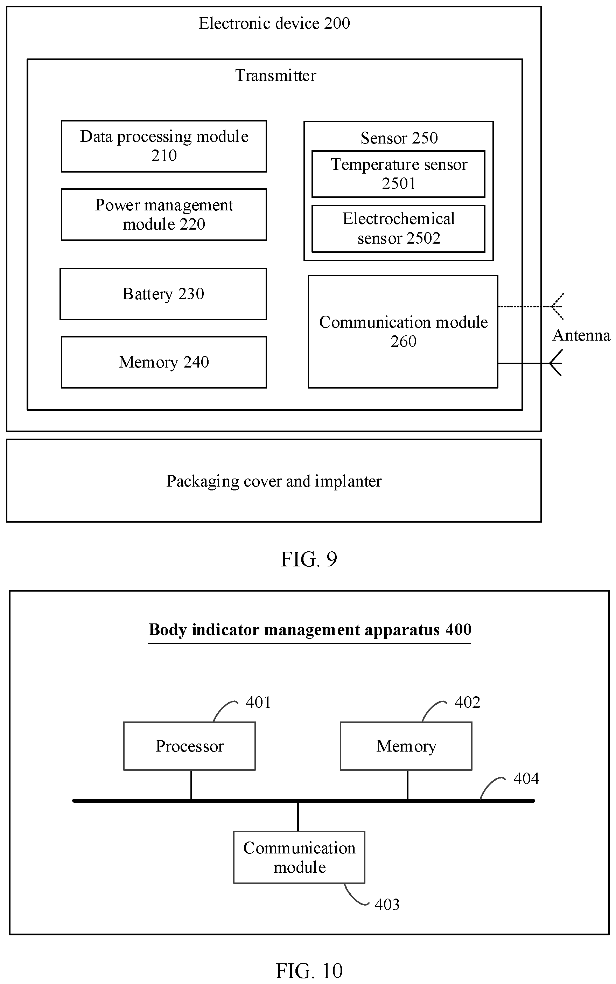

**FIG. 9** **is a diagram of a hardware structure of the electronic device 200 according to an embodiment of this application by using an example in which the electronic device 200 is a CGM device.**

As shown in FIG. 9, the electronic device 200 may include a transmitter, a packaging cover, and an implanter.

Before wearing, the packaging cover covers a transmitting outlet of the implanter, the transmitter is located in the implanter, and the implanter may include a transmitting button. When wearing the electronic device 200, the user may first remove the packaging cover, then align and attach the transmitting outlet of the implanter to a part of the user's body where the transmitter needs to be worn, for example, an upper arm or an abdomen, and then press or push the transmitting button to push out the transmitter in the implanter, so that the transmitter is attached to the skin. At the same time, a sensor equipped on the transmitter is implanted under the skin, so that the electronic device 200 can monitor a body indicator of the user based on data collected by the sensor.

The transmitter may include hardware such as a data processing module 210, a power management module 220, a battery 230, a memory 240, a sensor 250, and a communication module 260.

The data processing module 210 may include one or more processing units. For example, the data processing module 210 may include a modem processor, a digital signal processor, a controller, a baseband processor, a neural-network processing unit, and the like. Different processing units may be independent devices, or may be integrated into one or more processors. The data processing module 210 may also be referred to as a processor.

The memory 240 may be configured to store data collected by the sensor 250, and data obtained through calculation after the data processing module 210 processes the data collected by the sensor 250, for example, data of a body indicator of the user.

The sensor 250 may include one or more sensors, for example, a temperature sensor 2501 and an electrochemical sensor 2502.

The temperature sensor 2501 may be configured to detect a temperature. In some implementations, the electronic device 200 may determine a skin temperature of the user and/or an ambient temperature based on the temperature detected by the temperature sensor 2501.

The electrochemical sensor 2502 may be configured to detect a glucose concentration in blood, namely, a blood glucose value of the user. In some implementations, the electrochemical sensor 2502 may determine the glucose concentration by detecting oxygen consumption under catalysis of glucose oxidase or H₂O₂ generated by a glucose oxidation reaction in tissue fluid. In some embodiments, the electrochemical sensor 2502 connects the glucose oxidase to an electrode surface by using an electronic medium like a nanomaterial, metal osmium, ferrocene, or benzene quinone, then implements electron transfer through a series of oxidation-reduction reactions, and further determines the glucose concentration.

The communication module 260 may provide a wireless communication solution that is applied to the electronic device and that includes a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, or the like. The communication module 260 may be one or more components integrating at least one communication processor module. The communication module 260 receives an electromagnetic wave through the antenna, performs frequency modulation and filtering on an electromagnetic wave signal, and sends a processed signal to the data processing module 210. The communication module 260 may further receive a to-be-sent signal from the data processing module 210, perform frequency modulation and amplification on the signal, and convert the signal into an electromagnetic wave for radiation through the antenna.

**In some embodiments, the electronic device 200 may send a broadcast signal to surrounding devices by using the communication module 260, receive information that is sent by another device, for example, the electronic device 100, and that indicates that the electronic device 100 agrees to establish a communication connection, send, to the electronic device 100, body indicator data collected in real time, and the like.**

It may be understood that the structure shown in embodiments of this application does not constitute a specific limitation on the electronic device 200. In some other embodiments of this application, the electronic device 200 may include more or fewer components than those shown in the figure, or some components may be combined, or some components may be split, or different component arrangements may be used. The components shown in the figure may be implemented by hardware, software, or a combination of software and hardware.

In some implementations, the electronic device 200 may further include an activation circuit. The activation circuit may be configured to activate the electronic device 200. Further, the activation circuit may be configured to activate the electronic device 200 before or when the user wears the electronic device 200. For example, the activation circuit may include sensors like an optical sensor and a magnetic sensor. These sensors may be configured to sense a change of an ambient environment before the user wears the electronic device 200 or in a process of wearing the electronic device 200, to trigger the activation of the electronic device 200. The activation of the electronic device 200 may indicate that the battery 230 supplies power to the data processing module 210 through the power management module 220, so that the data processing module 210 is in a working state. Before the electronic device 200 is activated, the battery 230 cannot supply power to the data processing module 210 through the power management module 220, and the data processing module 210 cannot be in the working state.

In addition, a hardware structure of the electronic device 300 is similar to the hardware structure of the electronic device 200. Details are not described herein again.

**FIG. 10** **is a diagram of a structure of a body indicator management apparatus 400 according to an embodiment of this application.**

As shown in FIG. 10, the body indicator management apparatus 400 may include components such as a processor 401, a memory 402, and a communication module 403. These components may be connected through a bus 404 or in another manner. In FIG. 10, an example in which the components are connected through the bus is used. The bus 404 is configured to implement connection and communication between the processor 401, the memory 402, and the communication module 403.

The processor 401 may include one or more processing units. The processor 401 may be configured to provide a computing and control capability, to support running of the entire body indicator management apparatus 400.

The memory 402 may be configured to store various software programs and/or a plurality of groups of instructions. Specifically, the memory 402 may include a high-speed random access memory, and may also include a non-volatile memory, for example, one or more disk storage devices, flash memory devices, or other non-volatile solid-state storage devices.

The communication module 403 may be configured to communicate with another communication device. Specifically, the communication module 403 may include a communication interface. The communication interface may be a 3G communication interface, a long term evolution (LTE) (4G) communication interface, a 5G communication interface, a WLAN communication interface, a WAN communication interface, a human skin communication interface, or the like. In addition to the wireless communication interface, the body indicator management apparatus 400 may be further configured with a wired communication interface to support wired communication.

In this embodiment of this application, the body indicator management apparatus 400 may be the electronic device 100. The communication module 403 may be configured to obtain body indicator data collected by the electronic device 200 and body indicator data collected by the electronic device 300. The processor 401 may be configured to identify, based on the body indicator data collected by the electronic device 200 and the body indicator data collected by the electronic device 300, accuracy of the body indicator data collected by the electronic device 200 or the body indicator data collected by the electronic device 300. The memory 402 may be configured to store the body indicator data collected by the electronic device 200 and the body indicator data collected by the electronic device 300, and software or program code required by all or some functions of the electronic device 100 in the foregoing method embodiments.

It should be noted that the body indicator management apparatus 400 shown in FIG. 10 is merely an implementation of embodiments of this application. In an actual application, the body indicator management apparatus 400 may include more or fewer components than those shown in the figure, or some components may be combined, or different components may be deployed. This is not limited herein.

It should be understood that the steps in the foregoing method embodiments can be implemented by using a hardware integrated logical circuit in the processor, or by using instructions in a form of software. The steps of the methods disclosed with reference to embodiments of this application may be directly performed and completed by a hardware processor, or may be performed and completed by using a combination of hardware in the processor and a software module.

This application further provides an electronic device. The electronic device may include a memory and a processor. The memory may be configured to store a computer program. The processor may be configured to invoke the computer program in the memory, to enable the electronic device to perform the method performed by the electronic device 100 in any one of the foregoing embodiments.

This application further provides a chip system. The chip system includes at least one processor, configured to implement functions in the method performed by the electronic device 100 in any one of the foregoing embodiments.

In a possible design, the chip system further includes a memory, the memory is configured to store program instructions and data, and the memory is located inside or outside the processor.

The chip system may include a chip, or may include a chip and another discrete component.

Optionally, there may be one or more processors in the chip system. The processor may be implemented by using hardware, or may be implemented by using software. When the processor is implemented by using the hardware, the processor may be a logic circuit, an integrated circuit, or the like. When the processor is implemented by using the software, the processor may be a general-purpose processor, and is implemented by reading software code stored in the memory.

Optionally, there may also be one or more memories in the chip system. The memory may be integrated with the processor, or may be disposed separately from the processor. This is not limited in embodiments of this application. For example, the memory may be a non-transitory processor, for example, a read-only memory ROM. The memory and the processor may be integrated into a same chip, or may be separately disposed on different chips. A type of the memory and a manner of disposing the memory and the processor are not specifically limited in embodiments of this application.

For example, the chip system may be a field programmable gate array (field programmable gate array, FPGA), an application-specific integrated circuit (application specific integrated circuit, ASIC), a system on a chip (system on a chip, SoC), a central processing unit (central processing unit, CPU), a network processor (network processor, NP), a digital signal processor (digital signal processor, DSP), a micro controller unit (micro controller unit, MCU), a programmable logic device (programmable logic device, PLD), or another integrated chip.

This application further provides a computer program product. The computer program product includes a computer program (which may also be referred to as code or instructions). When the computer program is run, a computer is enabled to perform any method performed by the electronic device 100 in any one of the foregoing embodiments.

This application further provides a computer-readable storage medium. The computer-readable storage medium stores a computer program (which may also be referred to as code or instructions). When the computer program is run, a computer is enabled to perform any method performed by the electronic device 100 in any one of the foregoing embodiments.

It should be noted that the processor in embodiments of this application may be an integrated circuit chip, and has a signal processing capability. In an implementation process, steps in the foregoing method embodiments can be implemented by using a hardware integrated logical circuit in the processor, or by using instructions in a form of software. The processor may be a general-purpose processor, a digital signal processor (digital signal processor, DSP), an application-specific integrated circuit (Application-specific integrated circuit, ASIC), a field programmable gate array (field programmable gate array, FPGA) or another programmable logic device, a discrete gate or a transistor logic device, or a discrete hardware component. The processor may implement or perform the methods, the steps, and logical block diagrams that are disclosed in embodiments of this application. The general-purpose processor may be a microprocessor, or the processor may be any conventional processor or the like. The steps of the methods disclosed with reference to embodiments of this application may be directly performed and completed by a hardware decoding processor, or may be performed and completed by using a combination of hardware in the decoding processor and a software module. The software module may be located in a mature storage medium in the art, for example, a random access memory, a flash memory, a read-only memory, a programmable read-only memory, an electrically erasable programmable memory, or a register. The storage medium is located in the memory, and the processor reads information in the memory and completes the steps in the foregoing methods in combination with hardware of the processor.

In addition, an embodiment of this application further provides an apparatus. The apparatus may be specifically a component or a module, and the apparatus may include one or more processors and a memory that are connected to each other. The memory is configured to store a computer program. When the computer program is executed by one or more processors, the apparatus is enabled to perform the methods in the foregoing method embodiments.

The apparatus, the computer-readable storage medium, the computer program product, or the chip provided in embodiments of this application are all configured to perform the corresponding method provided above. Therefore, for beneficial effects that can be achieved, refer to beneficial effects in the corresponding methods provided above. Details are not described herein again.

The implementations of this application may be randomly combined to achieve different technical effect.

All or some of the foregoing embodiments may be implemented by using software, hardware, firmware, or any combination thereof. When software is used to implement the foregoing embodiments, all or some of embodiments may be implemented in a form of a computer program product. The computer program product includes one or more computer instructions. When the computer program instructions are loaded and executed on a computer, procedures or functions according to this application are all or partially generated. The computer may be a general-purpose computer, a dedicated computer, a computer network, or another programmable apparatus. The computer instructions may be stored in a computer-readable storage medium, or may be transmitted from a computer-readable storage medium to another computer-readable storage medium. For example, the computer instructions may be transmitted from a website, computer, server, or data center to another website, computer, server, or data center in a wired (for example, a coaxial cable, an optical fiber, or a digital subscriber line) or wireless (for example, infrared, radio, or microwave) manner. The computer-readable storage medium may be any usable medium accessible by the computer, or a data storage device, for example, a server or a data center, integrating one or more usable media. The usable medium may be a magnetic medium (for example, a floppy disk, a hard disk, or a magnetic tape), an optical medium (for example, a DVD), a semiconductor medium (for example, a solid state disk (solid state disk, SSD)), or the like.

Persons of ordinary skill in the art may understand that all or some of the procedures of the methods in embodiments may be implemented by a computer program instructing relevant hardware. The program may be stored in a computer-readable storage medium. When the program runs, the procedures of the method embodiments may be included. The foregoing storage medium includes any medium that can store program code, such as a ROM, a random access memory RAM, a magnetic disk, or an optical disc.

In conclusion, the foregoing descriptions are merely embodiments of the technical solutions of the present invention, but are not intended to limit the protection scope of the present invention. Any modification, equivalent replacement, improvement, or the like made according to the disclosure of the present invention shall fall within the protection scope of the present invention.

## Claims

1. A body indicator management method, wherein the method comprises:
obtaining, by a first device, first data, wherein the first data indicates a status of a body indicator of a user monitored by a second device at first time;
obtaining, by the first device, second data, wherein the second data indicates a status of the body indicator of the user monitored by a third device at the first time; and
identifying, by the first device, accuracy of the first data or the second data based on the first data and the second data.

2. The method according to claim 1, wherein after identifying, by the first device, the accuracy of the first data or the second data based on the first data and the second data, the method further comprises:
outputting, by the first device, first prompt information, wherein the first prompt information is used to give the user a prompt that the first data or the second data is inaccurate.

3. The method according to claim 1 or 2, wherein the method further comprises:
displaying, by the first device, a first curve, wherein the first curve comprises any one or more of the following: a curve segment drawn based on the first data, a curve segment drawn based on the second data, or a curve segment drawn based on the first data and the second data.

4. The method according to claim 3, wherein the first time comprises a first time period and a second time period, data in the first time period and data in the second time period that are comprised in the first data are accurate, data in the first time period comprised in the second data is inaccurate, and data in the second time period comprised in the second data is accurate; and
the curve segment drawn based on the first data and the second data comprises: a curve segment drawn based on the data in the first data in the first time period, and a curve segment drawn based on third data, wherein the third data is the data in the first data or the second data in the second time period, or the third data is data determined based on the data in the first data and the data in the second data in the second time period.

5. The method according to any one of claims 1 to 4, wherein the first time comprises the first time period and the second time period, the data in the second data in the first time period is inaccurate, the data in the second data in the second time period is accurate, and after identifying, by the first device, the accuracy of the first data or the second data based on the first data and the second data, the method further comprises:
displaying, by the first device, a first curve drawn based on the second data, wherein the first curve comprises a curve segment corresponding to the second time period, and does not comprise a curve segment corresponding to the first time period, or the first curve comprises a first curve segment corresponding to the first time period and a second curve segment corresponding to the second time period, and a display effect of the first curve segment and a display effect of the second curve segment are different.

6. The method according to claim 5, wherein the first curve comprises the curve segment corresponding to the second time period, and does not comprise the curve segment corresponding to the first time period; and after displaying, by the first device, the first curve drawn based on the second data, the method further comprises:
detecting, by the first device, a first operation, and updating the first curve, wherein the updated first curve comprises the curve segment corresponding to the first time period and the curve segment corresponding to the second time period.

7. The method according to claim 5 or 6, wherein the first device further displays first exception information, and the first exception information indicates a reason why the data in the second data in the first time period is inaccurate.

8. The method according to any one of claims 1 to 7, wherein after identifying, by the first device, the accuracy of the first data or the second data based on the first data and the second data, the method further comprises:
evaluating, by the first device, the body indicator of the user based on fourth data, wherein the fourth data comprises accurate data in the first data and the accurate data in the second data, and does not comprise inaccurate data in the first data and the inaccurate data in the second data.

9. The method according to any one of claims 1 to 8, wherein before obtaining, by the first device, the first data, the first device establishes a communication connection to the second device, and the method further comprises:
outputting, by the first device, second prompt information at second time, wherein the second prompt information is used to prompt the user to wear a device configured to monitor the body indicator, and a first preset duration is to elapse since the second time until the second device expires.

10. The method according to claim 9, wherein the first preset duration is greater than a first duration, and the first duration indicates an initialization duration of the device configured to monitor the body indicator.

11. The method according to any one of claims 1 to 10, wherein before obtaining, by the first device, the first data, the method further comprises:
after establishing the communication connection to the second device, outputting, by the first device after a second duration, data that is collected by the second device and that is used to reflect the body indicator of the user, wherein the second duration is determined based on the first duration and a duration from starting to wear the second device to establishing the communication connection to the first device by the second device, and the first duration indicates the initialization duration of the device configured to monitor the body indicator.

12. The method according to any one of claims 1 to 11, wherein wearing time of the second device is earlier than wearing time of the third device, the first time is in a first preset time period after the third device starts to be worn, and the first device identifies the accuracy of the second data based on the first data and the second data; and
if a probability that early sensitivity attenuation ESA occurs on the second device when the second device collects the first data is less than a first threshold, and a probability that early sensitivity attenuation occurs on the third device when the third device collects the second data is greater than a second threshold, the second data is inaccurate;
or
if a probability that early sensitivity attenuation occurs on the third device when the third device collects the second data is greater than a third threshold, the second data is inaccurate, wherein the probability that the early sensitivity attenuation occurs on the third device when the third device collects the second data is determined based on a probability that early sensitivity attenuation occurs on the second device when the second device collects the first data.

13. The method according to claim 12, wherein the first preset time period is preset by a developer or determined by the first device based on historically obtained body indicator data.

14. The method according to any one of claims 1 to 13, wherein the wearing time of the second device is earlier than the wearing time of the third device, the first time is in a second preset time period before the second device expires, and the first device identifies the accuracy of the first data based on the first data and the second data; and
if a probability that late sensor attenuation LSA occurs on the second device when the second device collects the first data is greater than a fourth threshold, and a probability that late sensor attenuation occurs on the third device when the third device collects the second data is less than a fifth threshold, the first data is inaccurate;
or
if a probability that late sensor attenuation occurs on the second device when the second device collects the first data is greater than a sixth threshold, the first data is inaccurate, wherein the probability that the late sensor attenuation occurs on the second device when the second device collects the first data is determined based on a probability that late sensor attenuation occurs on the third device when the third device collects the second data.

15. The method according to claim 14, wherein the second preset time period is preset by the developer or determined by the first device based on the historically obtained body indicator data.

16. The method according to any one of claims 1 to 15, wherein identifying, by the first device, the accuracy of the first data or the second data based on the first data and the second data specifically comprises:
if the first device identifies, based on the first data and the second data, that the second device is squeezed at the first time, determining, by the first device, that the first data is inaccurate.

17. The method according to any one of claims 1 to 16, wherein
the second device and the third device each are a continuous glucose monitoring CGM device, and the body indicator is blood glucose; or
the second device and the third device each are a continuous ketone monitoring CKM device, and the body indicator is blood ketone; or
the second device and the third device each are a continuous lactate monitoring CLM device, and the body indicator is lactate.

18. An electronic device, comprising a memory, one or more processors, and one or more programs, wherein when the one or more processors execute the one or more programs, the electronic device is enabled to implement the method according to any one of claims 1 to 17.

19. A computer-readable storage medium comprising instructions, wherein when the instructions are run on an electronic device, the electronic device is enabled to perform the method according to any one of claims 1 to 17.

20. A computer program product, comprising a computer program, wherein when the computer program is executed by a processor, the method according to any one of claims 1 to 17 is implemented.
